(19)

(11) **EP 3 186 221 B1**

(12) **EUROPÄISCHE PATENTSCHRIFT**

(45) Veröffentlichungstag und Bekanntmachung des Hinweises auf die Patenterteilung:
**23.02.2022 Patentblatt 2022/08**

(21) Anmeldenummer: **15757468.2**

(22) Anmeldetag: **25.08.2015**

(51) Internationale Patentklassifikation (IPC):
***C07C 209/48*** (2006.01) ***C07C 211/11*** (2006.01)
***C07C 211/09*** (2006.01)

(52) Gemeinsame Patentklassifikation (CPC):
(C-Sets verfügbar)
**C07C 209/48;** Y02P 20/582 (Forts.)

(86) Internationale Anmeldenummer:
**PCT/EP2015/069460**

(87) Internationale Veröffentlichungsnummer:
**WO 2016/030383 (03.03.2016 Gazette 2016/09)**

(54) **VERFAHREN ZUR HERSTELLUNG VON PRIMÄREN AMINEN UNTER VERWENDUNG EINES KOBALT-VOLLKONTAKTKATALYSATORS**

METHOD FOR THE PREPARATION OF PRIMARY AMINES USING A FULL CONTACT COBALT CATALYST

PROCÉDÉ DE FABRICATION D'AMINES PRIMAIRES À L'AIDE D'UN CATALYSEUR À COBALT EN CONTACT CONTINU

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.08.2014 EP 14182673**

(43) Veröffentlichungstag der Anmeldung:
**05.07.2017 Patentblatt 2017/27**

(73) Patentinhaber: **BASF SE**
**67056 Ludwigshafen am Rhein (DE)**

(72) Erfinder:
- **WIGBERS, Christof, Wilhelm**
  **35423 Lich (DE)**
- **MÄGERLEIN, Wolfgang**
  **67117 Limburgerhof (DE)**
- **KRUG, Thomas**
  **67550 Worms (DE)**
- **MELDER, Johann-Peter**
  **67459 Böhl-Iggelheim (DE)**
- **HEIDEMANN, Thomas**
  **68519 Viernheim (DE)**
- **STEIN, Bernd**
  **64665 Alsbach-Hähnlein (DE)**

(74) Vertreter: **BASF IP Association**
**BASF SE**
**G-FLP-C006**
**67056 Ludwigshafen (DE)**

(56) Entgegenhaltungen:
**EP-A2- 0 424 738**
**WO-A1-2006/077233**
**WO-A2-2010/089346**
**FR-A- 1 530 809**
**US-A- 5 756 845**
**US-A- 5 869 653**
**US-A1- 2010 036 168**
**US-A1- 2014 142 341**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**(Forts. nächste Seite)**

(52) Gemeinsame Patentklassifikation (CPC): (Forts.)

C-Sets
**C07C 209/48, C07C 211/09;**
**C07C 209/48, C07C 211/11;**
**C07C 209/48, C07C 211/12;**
**C07C 209/48, C07C 211/18**

**Beschreibung**

Die vorliegende Erfindung betrifft ein Verfahren zur Herstellung von primären Aminen, wobei ein Nitril in Gegenwart eines Kobalt-Vollkontaktkatalysators unter Erhalt des entsprechenden primären Amins hydriert wird. Die Hydrierung wird in einer Vorrichtung (V1), beispielsweise in einem Reaktor, durchgeführt. In diese Vorrichtung (V1) wird insbesondere während des Hydriervorgangs wiederkehrend oder kontinuierlich mindestens eine Verbindung (I) zugegeben, die auf Alkalimetallen basiert, beispielsweise wird als Verbindung (I) eine wässrige Lithiumhydroxid-Lösung zugegeben. Der Hydriervorgang findet in Abwesenheit von Ammoniak statt.

Bei der katalytischen Hydrierung von Nitrilen zu primären Aminen gemäß dem Stand der Technik kann die unerwünschte Bildung von sekundären und tertiären Aminen durch den Zusatz von Ammoniak oder Alkalihydroxiden weitgehend unterdrückt werden. Die hierbei verwendeten Katalysatoren enthalten als Hauptkomponente häufig Eisen, Kobalt und/oder Nickel. So kann z. B. Adipodinitril, ein mengenmäßig bedeutsames Nitril unter Hochdruckbedingungen in Gegenwart von elementarem Eisen und Ammoniak oder bei Mitteldruck in Gegenwart von Raney-Nickel und Natronlauge zu Hexamethylendiamin hydriert werden. Die Abtrennung und Rückführung von Ammoniak stellt einen nicht unwesentlichen Kostenfaktor für Investment und Energiekosten dar. Dies gilt auch für die Hydrierung von Zwischenprodukt-Nitrilen wie Isophoronnitril, Acetonitril und Dimethylaminopropionitril (DMAPN), zu den entsprechenden primären Aminen.

EP-A 913 388 beschreibt ein verbessertes Verfahren zur Hydrierung von Nitrilen zu primären Aminen, insbesondere von Dimethylaminopropionitril (DMAPN) zu Dimethylaminopropylamin (DMAPA). Hydriert wird in Gegenwart von suspendierten Kobaltschwamm- Katalysatoren wie Raney-Kobalt, Lithiumverbindungen und von Wasser ohne Zusatz von Ammoniak. Der Raney-Kobalt-Katalysator wird vor der Hydrierung mit 0,1 bis 100 mmol Lithiumhydroxid pro Gramm Kobaltschwamm-Katalysator behandelt. Nachteilig an der Fahrweise von EP-A 913 388 ist, dass die Abtrennung von Suspensionskatalysatoren aus Hydrierausträgen und ihre Rückführung in die Hydrierung oft mit Schwierigkeiten behaftet ist. Die in EP-A 913 388 beschriebenen Raney-Kobalt-Katalysatoren sind keine Kobalt-Vollkontaktkatalysatoren. Aufgrund der speziellen Herstellungsweise dieser Raney-Katalysatoren weisen diese eine sehr hohe Oberfläche auf.

WO 2003/070 688 beschreibt die Hydrierung von Nitrilen zu primären Aminen in Gegenwart von Nickel oder Kobalt-Katalysatoren, die ex situ mit Alkalicarbonaten oder Alkalihydrogencarbonaten, bevorzugt Kaliumcarbonat, vorbehandelt wurden. Unter ex situ ist dabei zu verstehen, dass der Katalysator außerhalb, insbesondere zeitlich vor der Hydrierungsreaktion von Nitril zu Amin modifiziert wurde. Als geeignete Katalysatoren werden insbesondere Raney-Nickel-Katalysatoren beschrieben, gegebenenfalls können auch Raney-Kobalt-Katalysatoren eingesetzt werden. Nirgendwo ist jedoch beschrieben, dass die Vorbehandlung mit den Alkaliverbindungen auch während der Hydrierung kontinuierlich oder wiederkehrend erfolgen kann. Weiterhin ist kein Hinweis enthalten, dass auch Lithiumverbindungen hierfür geeignet sind.

Aus WO 2007/104 663 A1 ist es auch bekannt, Lithium enthaltende Kobalt-Festbettkatalysatoren in Abwesenheit von Ammoniak für die Hydrierung von Nitrilen zu primären Aminen einzusetzen. Derartige Katalysatoren lassen sich durch Reduktion von Katalysator-Vorläufern der Formel $LiCoO_2$ mit Wasserstoff in einem Lösungsmittel wie Tetrahydrofuran herstellen. Solche Katalysator-Vorläufer sind auch durch Calcinieren von Gemischen aus Lithium- und Kobaltsalzen, wie z. B. Lithium- und Kobaltcarbonat, zugänglich. Allerdings ist in WO 2007/104 663 nirgendwo offenbart, dass bei der Hydrierung wiederkehrend oder kontinuierlich eine Verbindung (I) wie Lithiumhydroxid zugegeben werden kann.

Weiterhin ist bei den in WO 2007/104 663 beschriebenen, nach Reduktion bis zu 10 Gew.-% Lithium enthaltenden Kobalt-Katalysatoren nachteilig, dass im für die Hydrierung vorgesehenen Nitril und damit im Hydriergemisch enthaltenes Wasser zu einer Katalysator-Desaktivierung führt. Ein weiterer Nachteil ist, dass bei Verwendung von $LiCoO_2$ als Katalysator-Vorläufer bis zu 10 Gew.-% Lithium im Katalysator gebunden sind, ohne an die katalytisch aktive Zone zu gelangen, um dort katalytisch aktiv zu werden.

WO 2006/077233 A1, US 5,569,653 A und FR 1 530 809 A beschreiben die Hydrierung von Nitrilen zu primären Aminen in Anwesenheit eines Kobaltkatalysators. Es handelt sich dabei um Kobalt-Skelett-Katalysatoren (WO 2006/077233 A1) oder Katalysatoren des Raney-Typs (US 5,869,653 A, FR 1 530 809 A). Die Hydrierung wird in Anwesenheit einer Alkali-Metall-Verbindung im Reaktionsmedium durchgeführt.

WO 2010/089346 A2 und EP 0 424 738 A2 beschreiben die Hydrierung von Nitrilen zu primären Aminen unter Verwendung von kobaltenthaltenden Katalysatoren, welche weiterhin Elemente ausgewählt aus der Gruppe der Alkalimetalle, Erdalkalimetalle oder Seltenen Erden enthalten.

Die der vorliegenden Erfindung zugrundeliegende Aufgabe besteht in der Bereitstellung des neuen Verfahrens zur Herstellung von primären Aminen aus den entsprechenden Nitrilen unter Verwendung eines kobalthaltigen Katalysators.

Gelöst wird die Aufgabe durch ein Verfahren zur Herstellung von primären Aminen, das dadurch gekennzeichnet ist, dass mindestens ein Nitril in einer Vorrichtung (V1) in Gegenwart eines Kobalt-Vollkontaktkatalysators und in Abwesenheit von Ammoniak unter Erhalt von mindestens einem primären Amin hydriert wird, wobei in die Vorrichtung (V1) wiederkehrend oder kontinuierlich mindestens eine Verbindung (I) zugegeben wird und die Verbindung (I) mindestens eine Komponente ausgewählt aus Alkalimetall umfasst.

Durch das erfindungsgemäße Verfahren können in vorteilhafter Weise primäre Amine hergestellt werden, da Kobalt-Vollkontaktkatalysatoren, vorzugsweise in Form von Festbettkatalysatoren, eingesetzt werden. Dadurch ist auch eine Niederdruck-Hydrierung der entsprechenden Nitrile mit Wasserstoff in der Flüssigphase möglich. Weiterhin zeichnet sich das erfindungsgemäße Verfahren durch eine hohe Aktivität und Standzeit der eingesetzten Kobalt-Vollkontaktkatalysatoren aus. Aufgrund der kontinuierlichen oder zumindest wiederkehrenden Zugabe von mindestens einer Verbindung (I), insbesondere in Form von wässrigem Lithiumhydroxid, ist zudem eine hohe Ausbeute und Selektivität der gebildeten primären Amine festzustellen. Weitere Vorteile des erfindungsgemäßen Verfahrens sind darin zu sehen, dass das Verfahren auch in Gegenwart von Wasser, insbesondere unter Einsatz von wasserhaltigen Nitrilen, durchgeführt werden kann, und dass die Fahrweise ammoniakfrei ist, ohne dass es zu einem wesentlichen Absinken des Nitril-Umsatzes und der Selektivität an primären Aminen kommt.

Bei der Hydrierung von Nitrilen zu den entsprechenden Aminen ist es häufig erforderlich, einen hohen Umsetzungsgrad bezüglich der eingesetzten Nitrile zu erzielen, da nicht umgesetzte oder nur teilweise umgesetzte Nitrile nur schwer abzutrennen sind und in den Folgeanwendungen zu unerwünschten Eigenschaften, wie Geruch und Verfärbung, führen können.

Der Vorteil der vorliegenden Erfindung besteht somit auch darin, dass das erfindungsgemäße Verfahren die Hydrierung von Nitrilen bei hohen Umsätzen in hoher Selektivität und Ausbeute über lange Reaktionszeiten ermöglicht. Außerdem wird die Bildung von unerwünschten Nebenprodukten wie sekundären Aminen verringert.

Dadurch ist es möglich, die Hydrierung unter milderen Reaktionsbedingungen, insbesondere bei niedrigerem Druck und/oder bei niedrigerer Temperatur durchzuführen.

Somit ermöglicht die vorliegende Erfindung ein wirtschaftliches Verfahren zur Hydrierung. Insbesondere wird die Bildung von sekundären und tertiären Aminen, wie sie beispielsweise durch Reaktion von nicht umgesetztem Amin mit partiell hydriertem Nitril (Imin-Zwischenstufe) entstehen kann, verringert.

Insbesondere ermöglicht das erfindungsgemäße Verfahren die Herstellung von Isophorondiamin mit hoher Selektivität und Ausbeute. Insbesondere ist es möglich, den Gehalt an unerwünschtem Isophoronnitrilamin (IPNA) zu verringern. IPNA kann beispielsweise durch Reaktion von Isophoronnitril mit Ammoniak entstehen, welches zunächst zu Isophoronnitrilimin reagiert, welches dann bevorzugt mit Wasserstoff zu Isophoronnitrilamin reagiert.

Das erfindungsgemäße Verfahren dient ebenfalls bevorzugt zur Herstellung von 3-(Dimethylamino)-propylamin (DMAPA). Insbesondere ermöglicht das erfindungsgemäße Verfahren eine Verringerung des Gehalts an Bis-DMAPA. Als Zwischenprodukt wird es z. B. zur Herstellung von oberflächenaktiven Substanzen, Seifen, Kosmetik, Shampoos, Hygieneprodukten, Waschmittel und Pflanzenschutzmitteln verwendet. DMAPA wird auch zur Wasserbehandlung und als Polymerisationskatalysator für PU und Epoxy eingesetzt.

Ein Vorteil des erfindungsgemäßen Verfahrens gegenüber Verfahren des Standes der Technik, die auf Raney-Kobalt-Katalysatoren in Suspensionsfahrweise beruhen, ist auch darin zu sehen, dass solche Raney-Kobalt-Katalysatoren zum Einsatz in einer Festbettfahrweise prinzipiell ungeeignet sind. Der Einbau sowie der Ausbau dieser Raney-Kobalt-Katalysatoren sollte unter inerten Bedingungen erfolgen.

Das erfindungsgemäße Verfahren ist auch gegenüber Verfahren des Standes der Technik vorteilhaft, die auf die Verwendung sogenannter Monolith-Katalysatoren beruhen. Im Vergleich zum erfindungsgemäßen Verfahren unter Verwendung von Kobalt-Vollkontaktkatalysatoren ist die Verwendung von Monolith-Katalysatoren (Kobalt ist dort auf einen monolithischen Formkörper aufgebracht) bedeutend teurer in der Herstellung, ebenso ist der Einbau dieser Katalysatoren in einen konventionellen Reaktor schwieriger. Die Verwendung von Monolithkatalysatoren ist somit unökonomischer gegenüber dem erfindungsgemäßen Verfahren.

Auch gegenüber Verfahren, die auf die Verwendung von $LiCoO_2$-Katalysatoren beruhen, ist das erfindungsgemäße Verfahren vorteilhaft, weil bei den $LiCoO_2$-Katalysatoren bei zunehmender Betriebsdauer ein Aktivitätsverlust zu beobachten ist, da Lithium aus dem entsprechenden Katalysator auslaugt. Diese Art von Katalysator ist somit weniger gut geeignet, um Nitrilhydrierungen in Gegenwart von Wasser durchzuführen (leaching effect).

Nachfolgend wird das erfindungsgemäße Verfahren zur Herstellung von primären Aminen unter Verwendung eines Kobalt-Vollkontaktkatalysators näher definiert.

Im erfindungsgemäßen Verfahren können prinzipiell alle Nitrile, die dem Fachmann bekannt sind, eingesetzt werden. Erfindungsgemäß kann ein einziges Nitril oder aber auch ein Gemisch von zwei oder mehreren Nitrilen eingesetzt werden. Die eingesetzten Nitrile (Edukte) können dabei eine oder auch mehrere Nitrilfunktionen aufweisen. Erfindungsgemäß werden bei der Hydrierung die Nitrilfunktionen der eingesetzten Nitrile in die entsprechende Aminfunktion überführt. Vorzugsweise wird erfindungsgemäß aus jedem eingesetzten Nitril das entsprechende primäre Amin hergestellt, ohne dass dabei sonstige Komponenten (funktionelle Gruppen) des entsprechenden Nitrils chemisch verändert werden.

Vorzugsweise ist das Nitril ein aliphatisches Mono-, Di- oder Trinitril, ein cycloaliphatisches Mono- oder Dinitril, ein alpha-, beta- oder omega-Aminonitril oder ein Alkoxynitril.

Weiterhin bevorzugt werden aliphatische Mono-, Di- und/oder Trinitrile (linear oder verzweigt) mit 1 bis 30, insbesondere 2 bis 18 bzw. 2 bis 8 Kohlenstoffatomen oder cycloaliphatische Mono- und Dinitrile mit 6 bis 20, insbesondere 6 bis 12 Kohlenstoffatomen oder alpha-, beta- oder omega-Aminonitrile oder Alkoxynitrile mit 1 bis 30, insbesondere 2 bis 8 Kohlenstoffatomen im erfindungsgemäßen Verfahren eingesetzt.

Weiterhin können bevorzugt aromatische Nitrile mit 6 bis 18 Kohlenstoffatomen eingesetzt werden. Die oben genannten Mono-, Di- bzw. Trinitrile können ein- oder mehrfach substituiert sein.

Besonders bevorzugte Mononitrile sind Acetonitril zur Herstellung von Ethylaminen, Propionitril zur Herstellung von Propylaminen, Butyronitril zur Herstellung von Butylaminen, Lauronitril zur Herstellung von Laurylamin, Stearylnitril zur Herstellung von Stearylamin, N,N-Dimethylaminopropionitril (DMAPN) zur Herstellung von N,N-Dimethylaminopropylamin (DMAPA) und Benzonitril zur Herstellung von Benzylamin.

Besonders bevorzugte Dinitrile sind Adipodinitril (ADN) zur Herstellung von Hexamethylendiamin (HMD) und/oder 6-Aminocapronitril (ACN), 2-Methylglutarodinitril zur Herstellung von 2-Methylglutarodiamin, Succinonitril zur Herstellung von 1,4-Butandiamin und Korksäuredinitril zur Herstellung von Octamethylendiamin.

Besonders bevorzugte cyclische Nitrile sind Isophoronnitrilimin (IPNI) und oder Isophoronnitril (IPN) zur Herstellung von Isophorondiamin und Isophthalodinitril zur Herstellung von meta-Xylylendiamin.

Besonders bevorzugte $\beta$-Aminonitrile sind Aminopropionitril zur Herstellung von 1,3-Diaminopropan oder Additionsprodukte von Alkylaminen, Alkyldiaminen oder Alkanolaminen an Acrylnitril. So können Additionsprodukte von Ethylendiamin und Acrylnitril zu den entsprechenden Diaminen umgesetzt werden. Beispielsweise können 3-[2-Aminoethyl]aminopropionitril zu 3-(2-Aminoethyl)-aminopropylamin und 3,3'-(Ethylendiimino)bispropionitril bzw. 3-[2-(3-Amino-propylamino)-ethyl- amino]-propionitril zu N,N'-Bis-(3-aminopropyl)-ethylendiamin umgesetzt werden.

Besonders bevorzugte $\omega$-Aminonitrile sind Aminocapronitril zur Herstellung von Hexamethylendiamin und Caprolactam.

Weitere besonders bevorzugte Nitrile sind sogenannte "Strecker-Nitrile", wie Iminodiacetonitril zur Herstellung von Diethylentriamin und Aminoacetonitril (AAN) zur Herstellung von Ethylendiamin (EDA) und Diethylentriamin (DETA).

Ein bevorzugtes Trinitril ist Trisacetonitrilamin.

In einer besonders bevorzugten Ausführungsform wird N,N-Dimethylaminopropionitril (DMAPN) zur Herstellung von N,N-Dimethylaminopropylamin (DMAPA) in das erfindungsgemäße Verfahren eingesetzt.

In einer weiteren besonders bevorzugten Ausführungsform wird Isophoronnitrilimin zur Herstellung von Isophorondiamin in das erfindungsgemäße Verfahren eingesetzt und in einer weiteren besonders bevorzugten Ausführungsform wird Adipodinitril (ADN) zur Herstellung von Hexamethylendiamin (HMD) oder zur Herstellung von 6-Aminocapronitril (6-ACN) und HMD eingesetzt.

Die für die Hydrierung eingesetzten Nitrile können 0,01 bis 10, bevorzugt 0,1 bis 8, besonders bevorzugt 0,1 bis 5, ganz besonders bevorzugt 0,1 bis 3 Gew.-% Wasser enthalten. Dieses Wasser kann beispielsweise aus dem Verfahren zur Herstellung der verwendeten Nitrile stammen.

Die Hydrierung als solche, also die Reduktion der Nitrilfunktion des eingesetzten Nitriles unter Erhalt der entsprechenden Aminfunktion, kann prinzipiell nach allen dem Fachmann bekannten Methoden durchgeführt werden.

Als Reduktionsmittel für die Hydrierung können Wasserstoff oder ein Wasserstoff enthaltendes Gas verwendet werden. Der Wasserstoff kommt im Allgemeinen technisch rein zum Einsatz. Der Wasserstoff kann auch in Form eines Wasserstoff enthaltenden Gases, d. h. in Beimengungen mit anderen Inertgasen, wie Stickstoff, Helium, Neon, Argon oder Kohlendioxid, zum Einsatz kommen. Als Wasserstoff enthaltende Gase können beispielsweise Reformerabgase, Raffineriegase usw. verwendet werden, wenn und soweit diese Gase keine Kontaktgifte für die eingesetzten Hydrierkatalysatoren, wie zum Beispiel CO, enthalten. Bevorzugt wird jedoch reiner Wasserstoff bzw. im Wesentlichen reiner Wasserstoff in das Verfahren eingesetzt, beispielsweise Wasserstoff mit einem Gehalt von mehr als 99 Gew.-% Wasserstoff, bevorzugt mehr als 99,9 Gew.-% Wasserstoff, besonders bevorzugt mehr als 99,99 Gew.-% Wasserstoff, insbesondere mehr als 99,999 Gew.-% Wasserstoff.

Erfindungsgemäß wird die Hydrierung in einer Vorrichtung (V1) durchgeführt, in der sich neben den Edukten (Nitril sowie Reduktionsmittel) auch die weiter unten näher präzisierten weiteren Komponenten (Kobalt-Vollkontaktkatalysator sowie die Verbindung (1)) befinden.

Als Vorrichtung (V1) können prinzipiell alle dem Fachmann bekannten, für eine Hydrierung geeigneten Vorrichtungen verwendet werden. Bei der Vorrichtung (V1) kann es sich prinzipiell nur um eine einzige Vorrichtung handeln, es können aber auch zwei oder mehrere solcher Vorrichtungen parallel und/oder in Reihe geschaltet verwendet werden.

Das erfindungsgemäße Verfahren wird bevorzugt in einem Reaktor als Vorrichtung (V1) durchgeführt. Weiterhin ist es bevorzugt, dass in der Vorrichtung (V1), insbesondere im Reaktor, der Kobalt-Vollkontaktkatalysator als Festbett angeordnet ist.

In einer bevorzugten Ausführungsform umfasst die Festbettanordnung eine Katalysatorschüttung im eigentlichen Sinn, d. h. lose, geträgerte oder ungeträgerte Formkörper, die bevorzugt in der nachfolgend beschriebenen Geometrie oder Form vorliegen.

Dazu werden die Formkörper in den Reaktor eingebracht.

Damit die Formkörper in dem Reaktor verbleiben und nicht durch diesen hindurchfallen, wird üblicherweise ein Gitterboden oder ein gas- und flüssigkeitsdurchlässiges Blech eingesetzt, auf dem die Formkörper aufliegen.

Die Formkörper können sowohl am Eingang als auch am Ausgang des Reaktors von einem Inertmaterial umgeben sein. Als Inertmaterial werden in der Regel Formkörper eingesetzt, die eine ähnliche Geometrie aufweisen, wie die zuvor beschriebenen Katalysatorformkörper, sich jedoch in der Reaktion inert verhalten, z. B. Pallringe, Kugeln aus einem inerten Material (z.B. Keramik, Steatit, Aluminium).

Die Formkörper können aber auch mit Inertmaterial durchmischt werden und als Mischung in den Reaktor eingebracht werden.

Die Katalysatorschüttung (Formkörper + gegebenenfalls Inertmaterial) weist bevorzugt eine Schüttdichte (nach EN ISO 6) im Bereich von 0,1 bis 3 kg/l, bevorzugt von 1,5 bis 2,5 kg/l und insbesondere bevorzugt 1,7 bis 2,2 kg/l auf.

Der Differenzdruck über die Schüttung beträgt bevorzugt weniger als 1000 mbar/m, bevorzugt weniger als 800 mbar/m und besonders bevorzugt weniger als 700 mbar/m.

Bevorzugt liegt der Differenzdruck über die Schüttung im Bereich von 10 bis 1000 mbar/m, bevorzugt 50 bis 800 mbar/m, besonders bevorzugt 100 bis 700 mbar/m und insbesondere im Bereich von 200 bis 500 mbar/m.

Bei Rieselfahrweise (Durchflussrichtung der Flüssigkeit von oben nach unten) ergibt sich der Differenzdruck aus dem oberhalb der Katalysatorschüttung gemessenen Druck und dem unterhalb der Katalysatorschüttung gemessenen Druck.

Bei Sumpffahrweise (Durchflussrichtung der Flüssigkeit von unten nach oben) ergibt sich der Differenzdruck aus dem unterhalb der Katalysatorschüttung gemessenen Druck und dem oberhalb der Katalysatorschüttung gemessenen Druck.

Geeignete Festbettreaktoren sind beispielsweise in dem Artikel "Fixed-Bed Reactors" (Ullmann's Encyclopedia of Industrial Chemistry, veröffentlicht online: 15. Juni 2000, DOI: 10.1002/14356007.b04_199) beschrieben.

Bevorzugt wird das Verfahren in einem Schachtreaktor, Rohrbündelreaktor oder Rohrreaktor als Vorrichtung (V1) durchgeführt.

Besonders bevorzugt wird das Verfahren in einem Rohrreaktor durchgeführt.

Die Reaktoren können jeweils als einzelner Reaktor, als Serie von einzelnen Reaktoren und/oder in Form von zwei oder mehr parallelen Reaktoren eingesetzt werden.

Der spezielle Reaktoraufbau und die Durchführung der Reaktion können in Abhängigkeit von dem durchzuführenden Hydrierungsverfahren, den erforderlichen Reaktionszeiten und der Natur des eingesetzten Katalysators variieren.

Bevorzugt beträgt das Verhältnis von Höhe zu Durchmesser des Reaktors, insbesondere eines Rohrreaktors, 1 : 1 bis 500 : 1, besonders bevorzugt 2 : 1 bis 100 : 1 und insbesondere bevorzugt 5 : 1 bis 50 : 1.

Die Fließrichtung der Reaktanden (Edukte, Wasserstoff, gegebenenfalls flüssiger Ammoniak) ist in der Regel von oben nach unten bzw. von unten nach oben.

Besonders bevorzugt ist die Fließrichtung der Reaktanden (Edukte, Wasserstoff, gegebenenfalls flüssiger Ammoniak) von oben nach unten durch den Reaktor.

Die Katalysatorbelastung bei kontinuierlicher Fahrweise liegt typischerweise bei 0,01 bis 10 kg Edukt pro L Katalysator und Stunde, vorzugsweise von 0,2 bis 5 kg Edukt pro L Katalysator und Stunde, besonders bevorzugt von 0,2 bis 4 kg Edukt pro L Katalysator und Stunde, ganz besonders bevorzugt 0,2 bis 2 kg Edukt pro L Katalysator und Stunde.

Die Querschnittsbelastung liegt erfindungsgemäß im Bereich von 5 kg/($m^2$ s) bis 50 kg/($m^2$ s), bevorzugt im Bereich von 8 bis 25 kg/($m^2$ s), besonders bevorzugt im Bereich von 10 bis 20 kg/($m^2$ s) und insbesondere bevorzugt im Bereich von 12 bis 18 kg/($m^2$ s).

Die Querschnittsbelastung v [kg/($m^2$ s)] ist definiert als

$$v = \frac{Q}{A}$$

wobei Q die Durchflussmasse [kg/s] und A die Querschnittsfläche des leeren Reaktors ist [$m^2$].

Die Durchflussmasse Q ist wiederum definiert als die Summe der Massen aller zugeführten Eduktströme und Rückführströme. Wasserstoff, Kreisgase und eventuell zugeführte Inertgase werden nicht zur Berechnung der Durchflussmenge verwendet, da Wasserstoff, Kreisgase und Inertgase bei den üblichen Hydrierbedingungen in der Regel in der Gasphase vorliegen.

Um hohe Querschnittsbelastungen zu erzielen, wird bevorzugt ein Teil des Austrags (Teilaustrag) aus dem Hydrierreaktor als Rückführstrom in den Reaktor zurückgeführt (Umlaufstrom). Der Umlaufstrom kann dem Reaktor separat zugeführt werden, oder er kann besonders bevorzugt mit den zugeführten Edukten vermischt werden und zusammen mit diesem dem Reaktor wieder zugeführt werden.

Das Verhältnis von Umlaufstrom zum zugeführten Eduktstrom liegt bevorzugt im Bereich von 0,5 : 1 bis 250 :

1, besonders bevorzugt im Bereich von 1 : 1 bis 200 : 1, und insbesondere bevorzugt im Bereich von 2 : 1 bis 180 : 1. Wenn in das Verfahren kein Ammoniak zugeführt wird, dann liegt das Verhältnis von Umlaufstrom zum zugeführten Eduktstrom bevorzugt im oberen Bereich der voranstehend genannten Bereiche. Wenn hingegen in das Verfahren viel Ammoniak zugeführt wird, dann liegt das Verhältnis von Umlaufstrom zum zugeführten Eduktstrom bevorzugt im unteren Bereich der voranstehend genannten Bereiche.

In einer bevorzugten Ausführungsform der vorliegenden Erfindung wird aus der Vorrichtung (V1) ein Strom (S1) ausgeleitet, wobei der Strom (S1) das primäre Amin enthält und der Strom (S1) zumindest teilweise in die Vorrichtung (V1) rückgeleitet wird, vorzugsweise wird die Verbindung (I) zunächst in den rückgeleiteten Teil des Stromes (S1) zugegeben und als Bestandteil des Stroms (S1) in die Vorrichtung (V1) eingespeist, besonders bevorzugt wird die Verbindung (I) als wässrige Lösung in den rückgeleiteten Teil des Stroms (S1) zugegeben.

In einer weiteren bevorzugten Ausführungsform können hohe Querschnittsbelastungen erzielt werden, wenn die Reaktion in einem Reaktor mit schlanker Bauweise, insbesondere in einem Rohrreaktor mit schlanker Bauweise, durchgeführt wird.

Das Verhältnis von Höhe zu Durchmesser des Reaktors liegt deshalb, wie voranstehend beschrieben, bevorzugt im Bereich von 1 : 1 bis 500 : 1, besonders bevorzugt im Bereich von 2 : 1 bis 100: 1 und insbesondere bevorzugt im Bereich von 5 : 1 bis 50 : 1.

Kobalt-Vollkontaktkatalysatoren als solche sind dem Fachmann bekannt. Prinzipiell können im erfindungsgemäßen Verfahren alle bekannten Kobalt-Vollkontaktkatalysatoren eingesetzt werden. Vorzugsweise wird ein einziger Kobalt-Vollkontaktkatalysator eingesetzt, gegebenenfalls können auch Gemische aus zwei oder mehreren unterschiedlichen Kobalt-Vollkontaktkatalysatoren eingesetzt werden. Erfindungsgemäß ist es bevorzugt, dass der Kobalt-Vollkontaktkatalysator in der Vorrichtung (V1) als Festbettkatalysator verwendet wird.

Vorzugsweise werden Raney-Kobalt-Katalysatoren, auf Monoliten basierende kobalthaltige Katalysatoren oder kobalthaltige Katalysatoren, die auf einem $LiCoO_2$-Vorläufer beruhen, nicht als Kobalt-Vollkontaktkatalysatoren im Rahmen der vorliegenden Erfindung angesehen.

Die im erfindungsgemäßen Verfahren eingesetzten Kobalt-Vollkontaktkatalysatoren sind zunächst dadurch charakterisiert, dass sie

i) einen hohen Anteil an katalytisch aktiver Masse (Aktivkomponente) aufweisen,

ii) eine relativ geringe Oberfläche haben und/oder

iii) keinen oder nur einen relativ geringen Anteil an inertem Trägermaterial aufweisen.

Mit anderen Worten ausgedrückt bedeutet dies, dass die Kobalt-Vollkontaktkatalysatoren zu einem Großteil aus katalytisch aktiver Masse (Aktivkomponente) bestehen. Bei der katalytisch aktiven Masse handelt es sich insbesondere um Kobalt, wobei neben Kobalt auch noch weitere Elemente, insbesondere Metalle, als katalytisch aktive Masse enthalten sein können. In der Regel ist der Kobalt-Anteil der katalytisch aktiven Masse mindestens 55 Gew.-% und kann bis zu 100 Gew.-% betragen (die entsprechenden Katalysatoren enthalten nur Kobalt als katalytisch aktive Masse). Vorzugsweise beträgt der Kobaltanteil an der katalytisch aktiven Masse mindestens 80 Gew.-%, insbesondere mindestens 90 Gew.-%. Weiterhin ist es bevorzugt, dass die Obergrenze an katalytisch aktiver Masse in den Kobalt-Vollkontaktkatalysatoren 95 Gew.-% beträgt. Die restlichen 5 Gew.-% katalytisch aktiver Masse werden also von anderen Elementen gebildet, insbesondere von Promotoren, wie Mangan, Natrium und/oder Phosphor.

Weiterhin ist es erfindungsgemäß bevorzugt, dass der Kobalt-Vollkontaktkatalysator zu mindestens 70 Gew.-%, vorzugsweise zu mindestens 80 Gew.-%, besonders bevorzugt zu mindestens 90 Gew.-%, und ganz besonders bevorzugt zu mindestens 95 Gew.-% aus katalytisch aktiver Masse besteht.

Weiterhin ist es bevorzugt, dass der Kobalt-Vollkontaktkatalysator als katalytisch aktive Masse die Komponente i) und gegebenenfalls die Komponente ii) und/oder iii) umfasst, mit:

i) Kobalt (Co),

ii) gegebenenfalls mindestens ein weiteres Element ausgewählt aus Eisen (Fe), Nickel (Ni) Ruthenium (Ru), Rhodium (Rh), Palladium (Pd), Osmium (Os), Iridium (Ir) oder Platin (Pt), und/oder

iii) gegebenenfalls mindestens einen Promotor ausgewählt aus Chrom (Cr), Mangan (Mn), Molybdän (Mo), Titan (Ti), Zink (Zn), Zinn (Sn), Alkalimetalle, Erdalkalimetalle, Seltene Erdmetalle oder Phosphor (P).

Nach einer weiteren bevorzugten Ausführungsform der vorliegenden Erfindung besteht die katalytisch aktive Masse des Kobalt-Vollkontaktkatalysators aus 55 bis 98 Gew.-% Kobalt, 0,2 bis 15 Gew.-% Phosphor, 0,2 bis 15 Gew.-

% Mangan und 0,2 bis 15 Gew.-% Alkali (berechnet als CoO, $H_3PO_4$, $MnO_2$ oder Alkalioxid vor der Reduktion mit Wasserstoff). Vorzugsweise wird der Kobalt-Vollkontaktkatalysator hergestellt, indem die Katalysatormasse in einem ersten Schritt bei Endtemperaturen von 550 bis 750 °C und in einem zweiten Schritt bei Endtemperaturen von 800 bis 1000 °C calciniert wird. Solche Katalysatoren sind beispielsweise aus EP-A 0 636 409 bekannt.

Weiterhin ist es erfindungsgemäß bevorzugt, dass der Kobalt-Vollkontaktkatalysator

i) eine Oberfläche von maximal 50 $m^2/g$ aufweist, bevorzugt von maximal 40 $m^2/g$, besonders bevorzugt von maximal 30 $m^2/g$ und/oder
ii) der Anteil an Trägermaterial maximal 10 Gew.-% (bezogen auf die Katalysatorgesamtmasse), bevorzugt maximal 5 Gew.-%, vorzugsweise maximal 1 Gew.-%, ist, insbesondere ist der Kobalt-Vollkontaktkatalysator frei von Trägermaterial.

Sofern die im erfindungsgemäßen Verfahren eingesetzten Kobalt-Vollkontaktkatalysatoren ein Trägermaterial umfassen, handelt es sich dabei vorzugsweise um $ZrO_2$. Trägermaterialien, die auf Aluminiumoxiden basieren, werden erfindungsgemäß hingegen vorzugsweise vermieden.

In dem erfindungsgemäßen Verfahren können somit auch Katalysatoren eingesetzt werden, die durch Reduktion von sogenannten Katalysatorvorläufern hergestellt werden.

Der Katalysatorvorläufer enthält eine aktive Masse, die eine oder mehrere katalytisch aktive Komponenten, gegebenenfalls Promotoren und optional ein Trägermaterial enthält.

Bei den katalytisch aktiven Komponenten (Masse) handelt es sich um sauerstoffhaltige Verbindungen der oben genannten Metalle, beispielsweise um deren Metalloxide, Carbonate bzw. Hydroxide, wie CoO, NiO, CuO und/oder deren Mischoxide.

Im Rahmen dieser Anmeldung wird der Begriff "katalytisch aktive Komponenten" für oben genannte sauerstoffhaltige Metallverbindungen verwendet, soll aber nicht implizieren, dass diese sauerstoffhaltigen Verbindungen an sich bereits katalytisch aktiv sind. Die katalytisch aktiven Komponenten weisen in der Regel erst nach erfolgter Reduktion eine katalytische Aktivität in der erfindungsgemäßen Umsetzung auf.

Die Dotierelemente (Promotoren) sind bevorzugt in Mengen von nicht mehr als 10 Gew.-%, beispielsweise von 0,1 bis 10 Gew.-%, besonders bevorzugt in Mengen von 1 bis 5 Gew.-% enthalten, jeweils bezogen auf den jeweiligen Katalysatorvorläufer. Bevorzugte Katalysatoren enthalten 50 bis 99 Gew.-%, bevorzugt 70 bis 99 Gew.-%, besonders bevorzugt 90 bis 99 Gew.-% Kobalt.

Bevorzugt sind Katalysatorvorläufer, wie die in EP-A 0 636 409 offenbarten Oxidgemische, die vor der Reduktion mit Wasserstoff 55 bis 98 Gew.-% Co, berechnet als CoO, 0,2 bis 15 Gew.-% Phosphor, berechnet als $H_3PO_4$, 0,2 bis 15 Gew.-% Mangan, berechnet als $MnO_2$, und 0,2 bis 5,0 Gew.-% Alkali, berechnet als $M_2O$ (M=Alkali) enthalten und nach DE 34 03 377 A1 hergestellte Co/Mn/P-Katalysatoren oder in EP-A-0 742 045 offenbarte Oxidgemische, die vor der Reduktion mit Wasserstoff 55 bis 98 Gew.-% Co, berechnet als CoO, 0,2 bis 15 Gew.-% Phosphor, berechnet als $H_3PO_4$, 0,2 bis 15 Gew.-% Mangan, berechnet als $MnO_2$, und 0,05 bis 5 Gew.-% Alkali, berechnet als $M_2O$ (M=Alkali), enthalten oder die in EP-A 963 975 offenbarte Oxidgemische, die vor der Reduktion mit Wasserstoff 22 bis 40 Gew.-% ZrO2, 1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni : Cu-Verhältnis größer 1 ist, 15 bis 50 Gew.-% sauerstoffhaltige Verbindungen des Kobalts, berechnet als CoO, 0 bis 10 Gew.-% sauerstoffhaltige Verbindungen des Aluminiums und/oder Mangans, berechnet als Al2O3 bzw. MnO2, und keine sauerstoffhaltigen Verbindungen des Molybdäns enthält, beispielsweise der in loc. cit., Seite 17, offenbarte Katalysator A mit der Zusammensetzung 33 Gew.-% Zr, berechnet als ZrO2, 28 Gew.-% Ni, berechnet als NiO, 11 Gew.-% Cu, berechnet als CuO und 28 Gew.-% Co, berechnet als CoO, enthalten, oder in EP 445 589 B1 offenbarte Oxidgemische, die vor der Reduktion 65 Gew.-% CoO, 4,7 Gew.-% Mn3O4, 10 Gew.-% CaO oder 69 Gew.-% CoO, 4,9 Gew.-% Mn3O4, 5,5 Gew.-% NiO, 10,4 Gew.-% Fe2O3 und 10,0 Gew.-% CaO enthalten.

Als Formkörper eignen sich Formkörper mit beliebiger Geometrie bzw. Form. Bevorzugte Formen sind Tabletten, Ringe, Zylinder, Sternstränge, Strangformen, Wagenräder oder Kugeln, besonders bevorzugt sind Tabletten, Ringe, Zylinder, Strangformen, Kugeln oder Sternstränge. Ganz besonders bevorzugt sind Strangformen oder Tabletten.

Bei Kugeln beträgt der Durchmesser der Kugelform bevorzugt 10 mm oder weniger, besonders bevorzugt 4 mm oder weniger, ganz besonders bevorzugt 3 mm oder weniger und insbesondere bevorzugt 2,5 mm oder weniger.

In einer bevorzugten Ausführungsform liegt bei Kugeln der Durchmesser der Kugelform bevorzugt im Bereich von 0,1 bis 10 mm, besonders bevorzugt im Bereich von 0,5 bis 4 mm, ganz besonders bevorzugt im Bereich von 1 bis 3 mm und insbesondere besonders bevorzugt im Bereich von 1,5 bis 2,5 mm

Bei Strängen oder Zylindern liegt das Verhältnis von Länge : Durchmessers bevorzugt im Bereich von 1 : 1 bis 20 : 1, besonders bevorzugt 1 : 1 bis 14 : 1, ganz besonders bevorzugt im Bereich von 1 : 1 bis 10 : 1 und insbesondere bevorzugt im Bereich von 1 : 2 bis 6 : 1.

Der Durchmesser der Stränge oder Zylinder beträgt bevorzugt 10 mm oder weniger, besonders bevorzugt 5 mm oder weniger, ganz besonders bevorzugt 3 mm oder weniger und insbesondere bevorzugt 2, 5 mm oder weniger.

In einer bevorzugten Ausführungsform liegt der Durchmesser der Stränge oder Zylinder vorzugsweise im Bereich von 0,1 bis 10 mm, besonders bevorzugt im Bereich von 0,5 bis 3 mm, ganz besonders bevorzugt im Bereich von 1 bis 2,5 mm und insbesondere bevorzugt im Bereich von 1,5 bis 2,5 mm.

Bei Tabletten beträgt die Höhe h der Tablette vorzugsweise 10 mm oder weniger, besonders bevorzugt 4 mm oder weniger, ganz besonders bevorzugt 3 mm oder weniger und insbesondere bevorzugt 2,5 mm oder weniger.

In einer bevorzugten Ausführungsform liegt die Höhe h der Tablette bevorzugt im Bereich von 0,1 bis 10 mm, besonders bevorzugt im Bereich von 0,5 bis 4 mm, ganz besonders bevorzugt im Bereich von 1 bis 3 mm und insbesondere bevorzugt im Bereich von 1,5 bis 2,5 mm. Das Verhältnis von Höhe h (bzw. Dicke) der Tablette zum Durchmesser D der Tablette beträgt bevorzugt 1 : 1 bis 1 : 5, besonders bevorzugt 1 : 1 bis 1 : 2,5, ganz besonders bevorzugt 1 : 1 bis 1 : 2.

Bei allen anderen Geometrien weist der Katalysatorformkörper im erfindungsgemäßen Verfahren jeweils bevorzugt einen Äquivalentdurchmesser L = 1/a' von 2 mm oder weniger, besonders bevorzugt 1 mm oder weniger, ganz besonders bevorzugt 0,7 mm oder weniger, und insbesondere bevorzugt 0,5 mm oder weniger auf, wobei a' die externe Oberfläche per Volumeneinheit ($mm_s^2/mm^3$) ist, mit:

$$a' = \frac{A_p}{V_p}$$

wobei $A_p$ die externe Oberfläche des Formkörpers ($mm_s^2$) und $V_p$ das Volumen des Formkörpers ($mm^3$) ist.

In einer bevorzugten Ausführungsform weist der Katalysatorformkörper im erfindungsgemäßen Verfahren bei allen anderen Geometrien jeweils bevorzugt einen Äquivalentdurchmesser L = 1/a' im Bereich von 0,1 bis 2 mm, besonders bevorzugt im Bereich von 0,1 bis 0,7 mm, ganz besonders bevorzugt im Bereich von 0,2 bis 0,5 mm und insbesondere bevorzugt im Bereich von 0,3 bis 0,4 mm auf.

Die Oberfläche und das Volumen des Formkörpers ergeben sich aus den geometrischen Abmessungen des Formkörpers gemäß den bekannten mathematischen Formeln.

Das Volumen kann auch nach folgender Methode berechnet werden, bei der man:

1. die innere Porosität des Formkörpers bestimmt (z. B. über Messung der Wasseraufnahme in [ml/g Kat] bei Raumtemperatur und 1 bar Gesamtdruck),

2. die Verdrängung des Formkörpers beim Eintauchen in eine Flüssigkeit (z. B. durch Gasverdrängung mittels Helium-Pyknometer) bestimmt und

3. die Summe beider Volumina bildet.

Die Oberfläche kann auch nach folgender Methode theoretisch berechnet werden, bei der man eine Umhüllende des Formkörpers definiert, deren Kurvenradius max. 5 μm beträgt (um nicht die innere Porenoberfläche durch "Eindringen" der Umhüllenden in die Poren mitzunehmen) und die den Formkörper möglichst innig berührt (keine Schnittfläche mit dem Träger). Anschaulich würde das einer sehr dünnen Folie entsprechen, die man um den Formkörper legt und dann von innen ein Vakuum anlegt, so dass sich die Folie möglichst eng um den Formkörper legt.

Der eingesetzte Formkörper weist bevorzugt eine Schüttdichte (nach EN ISO 6) im Bereich von 0,1 bis 3 kg/l, bevorzugt von 1,5 bis 2,5 kg/l und insbesondere bevorzugt 1,7 bis 2,2 kg/l.

In einer bevorzugten Ausführungsform werden die Katalysatoren in Form von Formkörpern in das erfindungsgemäße Verfahren eingesetzt, die durch Tränkung (Imprägnierung) von Trägermaterialien hergestellt werden, die oben genannte Geometrie aufweisen oder die nach der Tränkung zu Formkörpern, die die oben genannte Geometrie aufweisen verformt werden.

Als Trägermaterialien kommen beispielsweise Kohlenstoff, wie Graphit, Ruß, Graphen, Kohlenstoffnanoröhrchen und/oder Aktivkohle, Aluminiumoxid (gamma, delta, theta, alpha, kappa, chi oder Mischungen daraus), Siliziumdioxid, Zirkoniumdioxid, Zeolithe, Alumosilicate oder deren Gemische in Betracht.

Die Tränkung der obengenannten Trägermaterialien kann nach den üblichen Verfahren erfolgen (A. B. Stiles, Catalyst Manufacture - Laboratory and Commercial Preparations, Marcel Dekker, New York, 1983), beispielsweise durch Aufbringung einer Metallsalzlösung in einer oder mehreren Tränkstufen. Als Metallsalze kommen in der Regel wasserlösliche Metallsalze, wie die Nitrate, Acetate oder Chloride der entsprechenden katalytisch aktiven Komponenten oder Dotierelemente in Betracht, wie Co-Nitrat oder Co-Chlorid. Im Anschluss wird das getränkte Trägermaterial in der Regel getrocknet und ggf. calciniert.

Die Calcinierung wird im Allgemeinen bei Temperaturen zwischen 300 und 800 °C, vorzugsweise 350 bis 600 °C, insbesondere bei 450 bis 550 °C ausgeführt.

Die Tränkung kann auch nach der sogenannten "incipient wetness-Methode" erfolgen, bei der das Trägermaterial entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu calcinieren. Die mehrstufige Tränkung ist vorteilhaft dann anzuwenden, wenn das Trägermaterial in größerer Menge mit Metallsalzen beaufschlagt werden soll.

Zur Aufbringung mehrerer Metallkomponenten auf das Trägermaterial, kann die Tränkung gleichzeitig mit allen Metallsalzen oder in beliebiger Reihenfolge der einzelnen Metallsalze nacheinander erfolgen.

Bevorzugt werden Trägermaterialien eingesetzt, die bereits die zuvor beschriebene bevorzugte Geometrie der Formkörper aufweisen.

Es ist jedoch auch möglich Trägermaterialien einzusetzen, die als Pulver oder Splitt vorliegen, und getränkte Trägermaterialien einer Formgebung zu unterziehen

So kann beispielsweise das imprägnierte und getrocknete bzw. calcinierte Trägermaterial konditioniert werden.

Die Konditionierung kann beispielsweise dadurch erfolgen, dass man das getränkte Trägermaterial durch Vermahlen auf eine bestimmte Korngröße einstellt. Nach der Vermahlung kann das konditionierte, getränkte Trägermaterial mit Formhilfsmitteln wie Graphit, oder Stearinsäure vermischt werden und zu Formkörpern weiterverarbeitet werden.

Gängige Verfahren der Formgebung sind beispielsweise in Ullmann’s Encyclopedia Electronic Release 2000, Kapitel: "Catalysis and Catalysts", Seiten 28 - 32 und von Ertl et al. (Ertl, Knözinger, Weitkamp, Handbook of Heterogenoeous Catalysis, VCH Weinheim, 1997, Seiten 98 ff.) beschrieben.

Gängige Verfahren der Formgebung sind beispielsweise Extrusion, Tablettieren, d.h. mechanisches Verpressen oder Pelletieren, d. h. Kompaktieren durch kreisförmige und/oder rotierende Bewegungen.

Durch den Prozess der Formgebung können Formkörper mit der oben genannten Geometrie erhalten werden.

Nach der Konditionierung bzw. Formgebung erfolgt in der Regel eine Temperung. Die Temperaturen bei der Temperung entsprechen üblicherweise den Temperaturen bei der Calcinierung.

In einer bevorzugten Ausführungsform werden Formkörper in das erfindungsgemäße Verfahren eingesetzt, die durch eine gemeinsame Fällung (Mischfällung) aller ihrer Komponenten hergestellt werden und die so ausgefällten Katalysatorvorläufer einer Formgebung unterzogen werden.

Dazu wird in der Regel eine lösliche Verbindung der entsprechenden aktiven Komponente, der Dotierelemente und gegebenenfalls eine lösliche Verbindung eines Trägermaterials in einer Flüssigkeit in der Wärme und unter Rühren so lange mit einem Fällungsmittel versetzt, bis die Fällung vollständig ist.

Als Flüssigkeit wird in der Regel Wasser eingesetzt.

Als lösliche Verbindung der aktiven Komponenten kommen üblicherweise die entsprechenden Metallsalze, wie die Nitrate, Sulfate, Acetate oder Chloride, der voranstehend genannten Metalle in Betracht.

Als lösliche Verbindungen eines Trägermaterials werden in der Regel wasserlösliche Verbindungen von Ti, Al, Zr, Si etc., beispielsweise die wasserlöslichen Nitrate, Sulfate, Acetate oder Chloride dieser Elemente, verwendet.

Als lösliche Verbindungen der Dotierelemente werden in der Regel wasserlösliche Verbindungen der Dotierelemente, beispielsweise die wasserlöslichen Nitrate, Sulfate, Acetate oder Chloride dieser Elemente, eingesetzt.

In einer weiteren, bevorzugten Ausführungsform können die Formkörper durch Auffällung hergestellt werden.

Unter Auffällung wird eine Herstellmethode verstanden, bei der ein schwer lösliches oder unlösliches Trägermaterial in einer Flüssigkeit suspendiert wird und nachfolgend lösliche Verbindungen, wie lösliche Metallsalze, der entsprechenden Metalloxide zugegeben werden, welche dann durch Zugabe eines Fällungsmittels auf den suspendierten Träger aufgefällt werden (z. B. beschrieben in EP-A2 1 106 600, Seite 4, und A. B. Stiles, Catalyst Manufacture, Marcel Dekker, Inc., 1983, Seite 15).

Als schwer- bzw. unlösliche Trägermaterialien kommen beispielsweise Kohlenstoffverbindungen, wie Graphit, Ruß und/oder Aktivkohle, Aluminiumoxid (gamma, delta, theta, alpha, kappa, chi oder Mischungen daraus), Siliziumdioxid, Zirkoniumdioxid, Zeolithe, Alumosilicate oder deren Gemische in Betracht.

Das Trägermaterial liegt in der Regel als Pulver oder Splitt vor.

Als Flüssigkeit, in der das Trägermaterial suspendiert wird, wird üblicherweise Wasser eingesetzt.

Als lösliche Verbindungen kommen die voranstehend genannten löslichen Verbindungen der aktiven Komponenten bzw. der Dotierelemente in Betracht.

Üblicherweise werden bei den Fällungsreaktionen die löslichen Verbindungen durch Zugabe eines Fällungsmittels als schwer- oder unlösliche, basische Salze gefällt.

Als Fällungsmittel werden bevorzugt Laugen, insbesondere Mineralbasen, wie Alkalimetallbasen eingesetzt. Beispiele für Fällungsmittel sind Natriumcarbonat, Natriumhydroxid, Kaliumcarbonat oder Kaliumhydroxid.

Als Fällungsmittel können auch Ammoniumsalze, beispielsweise Ammoniumhalogenide, Ammoniumcarbonat, Ammoniumhydroxid oder Ammoniumcarboxylate eingesetzt werden.

Die Fällungsreaktionen können z. B. bei Temperaturen von 20 bis 100 °C, besonders 30 bis 90 °C, insbesondere bei 50 bis 70 °C, durchgeführt werden.

Die bei den Fällungsreaktionen erhaltenen Niederschläge sind im Allgemeinen chemisch uneinheitlich und enthalten in der Regel Mischungen der Oxide, Oxidhydrate, Hydroxide, Carbonate und/oder Hydrogencarbonate der eingesetzten Metalle. Es kann sich für die Filtrierbarkeit der Niederschläge als günstig erweisen, wenn sie gealtert werden, d. h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden üblicherweise verarbeitet, indem sie gewaschen, getrocknet, calciniert und konditioniert werden.

Nach dem Waschen werden die Niederschläge im Allgemeinen bei 80 bis 200 °C, vorzugsweise 100 bis 150 °C, getrocknet und anschließend calciniert.

Die Calcinierung wird im Allgemeinen bei Temperaturen zwischen 300 und 800°C, vorzugsweise 350 bis 600 °C, insbesondere bei 450 bis 550 °C ausgeführt.

Nach der Calcinierung werden die durch Fällungsreaktionen erhaltenen pulverförmigen Katalysatorvorläufer üblicherweise konditioniert.

Die Konditionierung kann beispielsweise dadurch erfolgen, dass man den Fällungskatalysator durch Vermahlen auf eine bestimmte Korngröße einstellt

Nach der Vermahlung kann der durch Fällungsreaktionen erhaltene Katalysatorvorläufer mit Formhilfsmitteln wie Graphit, oder Stearinsäure vermischt werden und zu Formkörpern weiterverarbeitet werden.

Gängige Verfahren der Formgebung sind beispielsweise in Ullmann's Encyclopedia Electronic Release 2000, Kapitel: "Catalysis and Catalysts", Seiten 28 - 32, und von Ertl et al. (Ertl, Knözinger, Weitkamp, Handbook of Heterogenoeous Catalysis, VCH Weinheim, 1997, Seiten 98 ff.) beschrieben.

Gängige Verfahren der Formgebung sind beispielsweise Extrusion, Tablettieren, d. h. mechanisches Verpressen oder Pelletieren, d.h. Kompaktieren durch kreisförmige und/oder rotierende Bewegungen.

Durch den Prozess der Formgebung können Formkörper mit der oben genannten Geometrie erhalten werden.

Nach der Konditionierung bzw. Formgebung erfolgt in der Regel eine Temperung. Die Temperaturen bei der Temperung entsprechen üblicherweise den Temperaturen bei der Calcinierung.

Formkörper, die durch Tränkung oder Fällung hergestellt wurden, enthalten die katalytisch aktiven Komponenten nach erfolgter Calcinierung in der Regel in Form ihrer sauerstoffhaltigen Verbindungen, beispielsweise als deren Metalloxide bzw. Hydroxide, wie CoO, NiO, CuO und/oder deren Mischoxide (Katalysatorvorläufer).

Die Katalysatorvorläufer, die wie voranstehend beschrieben durch Imprägnierung oder Fällung hergestellt wurden, werden im Allgemeinen nach der Calcinierung bzw. Konditionierung reduziert. Durch die Reduktion wird der Katalysatorvorläufer in der Regel in seine katalytisch aktive Form umgewandelt.

Die Reduktion des Katalysatorvorläufers kann bei erhöhter Temperatur in einem bewegten oder unbewegten Reduktionsofen durchgeführt werden.

Als Reduktionsmittel wird üblicherweise Wasserstoff oder ein Wasserstoff enthaltendes Gas eingesetzt.

Der Wasserstoff kommt im Allgemeinen technisch rein zum Einsatz. Der Wasserstoff kann auch in Form eines Wasserstoff enthaltendem Gases, d.h. in Beimengungen mit anderen Inertgasen, wie Stickstoff, Helium, Neon, Argon oder Kohlendioxid zum Einsatz kommen. Der Wasserstoffstrom kann auch als Kreisgas in die Reduktion zurückgeführt werden, gegebenenfalls vermischt mit Frisch- Wasserstoff und gegebenenfalls nach Entfernen von Wasser durch Kondensation.

Die Reduktion des Katalysatorvorläufers erfolgt bevorzugt in einem Reaktor, in dem die Formkörper als Festbett angeordnet sind. Besonders bevorzugt erfolgt die Reduktion des Katalysatorvorläufers in demselben Reaktor in dem die nachfolgende Umsetzung der Nitrile mit Wasserstoff erfolgt.

Weiterhin kann die Reduktion des Katalysatorvorläufers in einem Wirbelschichtreaktor in der Wirbelschicht erfolgen.

Die Reduktion des Katalysatorvorläufers erfolgt in der Regel bei Reduktionstemperaturen von 50 bis 600 °C, insbesondere von 100 bis 500 °C, besonders bevorzugt von 150 bis 450 °C.

Der Wasserstoffpartialdruck beträgt in der Regel von 1 bis 300 bar, insbesondere von 1 bis 200 bar, besonders bevorzugt von 1 bis 100 bar, wobei sich die Druckangaben hier und im Folgenden auf den absolut gemessenen Druck beziehen.

Die Dauer der Reduktion beträgt bevorzugt 1 bis 20 Stunden, und besonders bevorzugt 5 bis 15 Stunden.

Während der Reduktion kann ein Lösungsmittel zugeführt werden, um entstehendes Reaktionswasser abzuführen und/oder um beispielsweise den Reaktor schneller aufheizen zu können und/oder während der Reduktion die Wärme besser abführen zu können. Das Lösungsmittel kann hierbei auch überkritisch zugeführt werden.

Als geeignete Lösungsmittel können die zuvor beschriebenen Lösungsmittel eingesetzt werden. Bevorzugte Lösungsmittel sind Wasser oder Ether wie Methyl-tert.-butylether, Ethyl-tert.-butylether, Dioxan oder Tetrahydrofuran. Besonders bevorzugt sind Wasser oder Tetrahydrofuran. Als geeignete Lösungsmittel kommen ebenfalls geeignete Mischungen in Betracht.

Der so erhaltene Formkörper kann nach der Reduktion unter inerten Bedingungen gehandhabt werden. Bevorzugt kann der Formkörper unter einem Inertgas wie Stickstoff gehandhabt und gelagert werden oder unter einer inerten Flüssigkeit, zum Beispiel einem Alkohol, Wasser oder dem Produkt der jeweiligen Reaktion, für die der Katalysator eingesetzt wird. Gegebenenfalls muss der Katalysator vor Beginn der eigentlichen Reaktion dann von der inerten Flüssigkeit befreit werden.

Die Lagerung des Katalysators unter inerten Substanzen ermöglicht eine unkomplizierte und ungefährliche Handhabung und Lagerung des Formkörpers.

Der Formkörper kann nach der Reduktion aber auch mit einem Sauerstoff enthaltenden Gasstrom wie Luft oder einem Gemisch von Luft mit Stickstoff in Kontakt gebracht werden.

Dadurch wird ein passivierter Formkörper erhalten. Der passivierte Formkörper weist im Allgemeinen eine schützende Oxidschicht auf. Durch diese schützende Oxidschicht wird die Handhabung und Lagerung des Katalysators vereinfacht, so dass beispielsweise der Einbau des passivierten Formkörpers in den Reaktor vereinfacht wird. Ein passivierter Formkörper wird bevorzugt vor dem Inkontaktbringen mit den Edukten wie oben beschrieben durch Behandlung des passivierten Katalysators mit Wasserstoff oder einem Wasserstoff enthaltenden Gas reduziert. Die Reduktionsbedingungen entsprechen im Allgemeinen den Reduktionsbedingungen, die bei der Reduktion der Katalysatorvorläufer angewandt werden. Durch die Aktivierung wird in der Regel die schützende Passivierungsschicht aufgehoben. Besonders bevorzugt wird die mindestens eine Verbindung (I) wiederkehrend oder kontinuierlich während der Hydrierung in die Vorrichtung (V1) zugegeben.

In die Vorrichtung (V1) - also die Hydrierung - wird erfindungsgemäß wiederkehrend oder kontinuierlich mindestens eine Verbindung (I) zugegeben. Die Verbindung (I) umfasst mindestens eine Komponente ausgewählt aus Alkalimetallen. Die Verbindung (I) als solche ist dem Fachmann bekannt. Vorzugsweise wird eine Verbindung (I) wiederkehrend oder kontinuierlich in die Vorrichtung (V1) zugegeben, gegebenenfalls können auch Gemische aus zwei oder mehreren Verbindungen (I) zugegeben werden. Weiterhin ist es möglich, dass sich bereits vor Beginn der Hydrierung mindestens eine Verbindung (I) in der Vorrichtung (V1) befindet, vorzugsweise wird die Verbindung (I) aber erst nach Beginn der Hydrierung in das erfindungsgemäße Verfahren zugegeben.

Die Zugabe von mindestens einer Verbindung (I) in die Vorrichtung (V1) kann wie vorstehend bereits ausgeführt wiederkehrend oder kontinuierlich erfolgen. Dabei kann die Verbindung (I) in flüssiger oder fester Form zugegeben werden. Dabei ist auch festzuhalten, dass die Verbindung (I) nicht direkt in die Vorrichtung (V1) zugegeben werden muss, sondern die Verbindung (I) kann zunächst auch in einer anderen Vorrichtung, beispielsweise in einer Kontaktvorrichtung (V2), einer oder mehreren der an der Hydrierung beteiligten Komponenten zugegeben werden. Aus dieser anderen Vorrichtung wird die Verbindung (I) in die Vorrichtung (V1) geführt (mittelbare Zugabe die Verbindung (I) nach (V1)). Das (Über-)Führen oder (Über-)Leiten der Verbindung (I) aus der anderen Vorrichtung in die Vorrichtung (V1) erfolgt nach den dem Fachmann bekannten Methoden, beispielsweise unter Verwendung von Pumpen. Insbesondere kann die Verbindung (I) wie vorstehend bereits ausgeführt einem rückgeleiteten Teil des Stroms (S1) zugegeben werden.

Unter einer "kontinuierlichen Zugabe" der Verbindung (I) wird im Rahmen der vorliegenden Erfindung verstanden, dass die entsprechende Zugabe über einen längeren Zeitraum, vorzugsweise über mindestens 50 %, mehr bevorzugt über mindestens 70 %, noch mehr bevorzugt über mindestens 90 %, insbesondere über die gesamte Reaktionsdauer erfolgt. Vorzugsweise wird die kontinuierliche Zugabe so durchgeführt, dass die entsprechende Vorrichtung (V1) zum Eintrag (Zugabe) der Verbindung (I) (z. B. eine Zellenradschleuse) über die vorgenannten Zeiträume in Betrieb ist.

Unter einer "wiederkehrenden Zugabe" der Verbindung (I) wird im Rahmen der vorliegenden Erfindung verstanden, dass die entsprechende Zugabe in regelmäßigen oder unregelmäßigen Zeitabständen erfolgt. Vorzugsweise wird die entsprechende Zugabe durch das Eintreten einer weiter unten beschriebenen Zugabebedingung veranlasst, insbesondere im Zusammenhang mit der Konzentration der Verbindung (I) im Austrag aus der Vorrichtung (V1). Die zeitlichen Abstände zwischen den einzelnen Zugaben betragen mindestens 1 h, vorzugsweise mindestens einen Tag. Im Rahmen der vorliegenden Erfindung werden unter dem Begriff "wiederkehrend" weiterhin mindestens zwei, beispielsweise 3, 4, 5, 10 oder auch 100 einzelne Zugaben verstanden. Die konkrete Anzahl der Einzelzugaben hängt von der Betriebsdauer ab. Diese geht idealerweise gegen Unendlich.

In anderen Worten ausgedrückt wird unter einer wiederkehrenden Zugabe der Verbindung (I) im Rahmen der vorliegenden Erfindung die zeitlich voneinander abgegrenzte Zugabe mehrerer Chargen an Verbindung (I) verstanden. Die Zugabe einer einzelnen Charge kann von mehreren Sekunden bis zu mehreren Minuten dauern, gegebenenfalls sind auch etwas längere Zeiträume denkbar. Erfindungsgemäß ist der zeitliche Abstand zwischen der jeweiligen Zugabe einer einzelnen Charge mindestens zehnfach so groß wie die Dauer der Zugabe der entsprechenden Charge. Gegebenenfalls kann im Rahmen der vorliegenden Erfindung die Ausführungsform einer "wiederkehrenden Zugabe" mit der Ausführungsform einer "kontinuierlichen Zugabe" auch miteinander kombiniert werden.

Die genaue Dosierung der wiederkehrenden oder kontinuierlichen Zugabe von mindestens einer Verbindung (I) wird nachfolgend anhand der Zugabe von Lithiumhydroxid exemplarisch verdeutlicht. Die Ausführungen gelten aber auch sinngemäß für sämtliche andere Verbindungen, die unter die Definition der Verbindung (I) der vorliegenden Erfindung fallen.

Die Lithium-Konzentration im Austrag sollte einen gewissen Schwellenwert nicht unterschreiten. Das Unterschreiten ist ein Indikator, dass Lithium aus dem Katalysator ausgelaugt wurde.

Die Lithium-Konzentration im Austrag sollte auch nicht zu hoch sein, da ansonsten der Katalysator seine Aktivität verliert (Vergiftung).

Die Dosierung kann kontinuierlich oder wiederkehrend (periodisch) erfolgen. Wesentlich ist, dass die Lithium-Konzentration im Austrag/Reaktionsgemisch nicht so hoch wird, dass es zu Ablagerungen kommt, aber so hoch ist, dass die Konzentration auf dem Katalysator erhalten bleibt.

Insbesondere gelten an der Zudosierungsstelle noch besondere Anforderungen:

- Die Lithium-Konzentration der zudosierten Lösung darf nicht zu hoch sein, da es sonst zu Ablagerungen/Verstopfungen im Reaktor kommt.

- Die Einspeisung sollte an einer Stelle des Reaktors vorgenommen werden, an der eine hohe Strömungsgeschwindigkeit herrscht, damit das LiOH sich gut mit dem Reaktionsgemisch vermischt. LiOH hat eine geringe Löslichkeit in Wasser und in den meisten organischen Lösungsmitteln.

Als Verbindungen (I) der Alkalimetalle kommen Li-, Na-, K-, Rb- und Cs-Verbindungen in Frage.

Bevorzugte Verbindungen (I) der Alkalimetalle sind Lithium, Kalium und Caesium.

Weiterhin ist es bevorzugt, dass die Verbindung (I) in Form eines Oxids, eines Hydroxids und/oder eines Salzes eingesetzt wird und/oder dass die Verbindung (I) als wässrige Lösung eingesetzt wird. Als Salze kommen z.B. Nitrate, Carbonate, Hydrogencarbonate, Phosphate, Carboxylate wie z.B. Formiate und Acetate in Frage. Bevorzugt sind Hydroxide, die im Rahmen der vorliegenden Erfindung nicht unter den Begriff "Salze" fallen.

Weiterhin ist es bevorzugt, dass eine wässrige Lösung eines Oxids, eines Hydroxids oder eines Salzes des Lithiums, Kaliums und/oder Caesiums verwendet wird.

Ganz besonders bevorzugt sind wässrige Lösungen von Verbindungen des Lithiums wie z.B. Lithiumhydroxid, Lithiumcarbonat, Lithiumphosphat, Lithiumnitrat, Lithiumformiat, Lithiumacetat. Bevorzugt ist hierbei Lithiumhydroxid, insbesondere bevorzugt ist also eine wässrige Lösung von Lithiumhydroxid.

Als Lösungsmittel für Alkaliverbindungen können grundsätzlich organische Lösungsmittel, Wasser oder Gemische dieser Lösungsmittel mit Wasser verwendet werden. Bevorzugt sind die Lösungsmittel, die auch als Lösungsmittel für die eingesetzten Nitrile weiter unten genannt sind. Sie müssen unter den Hydrierbedingungen stabil sein und die Alkaliverbindungen in ausreichendem Maße lösen.

Bevorzugt sind daher $C_1$- bis $C_4$-Alkohole, Wasser oder Gemische dieser Verbindungen. Ganz besonders bevorzugt ist Wasser.

Weiterhin ist es erfindungsgemäß bevorzugt, dass

i) das zur Hydrierung eingesetzte Nitril 0,01 bis 10 Gew.-% Wasser enthält, und/oder

ii) die Verbindung (I) in die Vorrichtung (V1) zugegeben wird, sobald der Anteil an als Nebenprodukt gebildetem sekundären Amin größer ist als 0,5 GC-Fl%, und/oder

iii) in die Vorrichtung (V1) dem Hydriergemisch über die Zugabe der Verbindung (I) 0,01 bis 500 ppm Alkalimetall, bezogen auf g-Atome Nitril in der Vorrichtung (V1), zugegeben werden, vorzugsweise werden in die Vorrichtung (V1) dem Hydriergemisch über die Zugabe der Verbindung (I) 0,01 bis 500 ppm Alkalimetall, bezogen auf g-Atome Nitril in der Vorrichtung (V1), zugegeben, sobald der Anteil an als Nebenprodukt gebildetem sekundären Amin in der Vorrichtung (V1) größer ist als 0,5 GC-Fl%, bei einer Katalysatorbelastung von 0,01 bis 10 kg Edukt pro L Katalysator und Stunde.

Unter "g-Atome Nitril in der Vorrichtung (V1)" wird im Rahmen der vorliegenden Erfindung die Gesamtmenge (das Gesamtgewicht) in Gramm (g) an Nitril in der Vorrichtung (V1) verstanden.

Somit ist es erfindungsgemäß bevorzugt, dass in die Vorrichtung (V1) dem Hydriergemisch über die Zugabe der Verbindung (I) 0,01 bis 500 ppm Alkalimetall, bezogen auf das Gesamtgewicht in Gramm an Nitril in der Vorrichtung (V1), zugegeben werden, vorzugsweise werden in die Vorrichtung (V1) dem Hydriergemisch über die Zugabe der Verbindung (I) 0,01 bis 500 ppm Alkalimetall, bezogen auf das Gesamtgewicht in Gramm an Nitril in der Vorrichtung (V1), zugegeben, sobald der Anteil an als Nebenprodukt gebildetem sekundärem Amin in der Vorrichtung (V1) größer ist als 0,5 GC-Fl%, bei einer Katalysatorbelastung von 0,01 bis 10 kg Edukt pro L Katalysator und Stunde.

Verfahren zur Bestimmung der GC-Fl% (GC-Flächen %) sind dem Fachmann als solche bekannt. Vorzugsweise werden die GC-Fl% mit folgenden Parametern bestimmt: GC-Säule: 60 m CP Volamine/WCOT fused Silica 0,32; Temperaturprogramm: 50 °C - 10 min - 15 °C/min - 240 °C - 30 min.

Die Hydrierung kann erfindungsgemäß diskontinuierlich, halbkontinuierlich, bevorzugt aber kontinuierlich durchgeführt werden.

Die Hydrierung wird in der Regel bei einem Druck von 1 bis 300 bar, insbesondere von 5 bis 150 bar, bevorzugt von 10 bis 100 bar und besonders bevorzugt von 15 bis 60 bar durchgeführt. Ganz besonders bevorzugt wird die Hydrierung bei einem Druck von weniger als 60 bar als Niederdruck-Verfahren ausgeführt

Die Temperatur liegt in der Regel in einem Bereich von 25 bis 300 °C, insbesondere von 50 bis 200 °C, bevorzugt von 70 bis 150 °C, besonders bevorzugt von 80 bis 140 °C.

Das molare Verhältnis von Wasserstoff zu eingesetzten Nitril beträgt in der Regel 2 : 1 bis 25 : 1, vorzugsweise 2 : 1 bis 10 : 1. Der Wasserstoff kann als Kreisgas in die Reaktion zurückgeführt werden.

Dabei werden die Reaktionsbedingungen bevorzugt so gewählt, dass die eingesetzten Nitrile und zugegebene Flüssigkeiten in der Regel in der Flüssigphase vorliegen und nur der eingesetzte Wasserstoff bzw. Inertgase unter den genannten Reaktionsbedingungen in der Gasphase vorliegen.

Das Ergebnis der Hydrierung kann, vor allem bei kontinuierlicher Durchführung, analytisch, z. B. gaschromatographisch, verfolgt werden. Falls die Analyse ein Absinken der Selektivität an primärem Amin unter Bildung von sek. Amin, z.B. Bis-DMAPA, als Nebenprodukt offenbart, kann dieses Absinken vorzugsweise durch Zugabe der genannten Lösungen rückgängig gemacht werden.

Die Verbindung (I) wird zudosiert, sobald der Anteil an gebildetem sek. Amin größer ist als 0,5 GC-Fl%, bevorzugt größer ist als 1 GC-Fl%, besonders bevorzugt größer ist als 2 GC-Fl%, ganz besonders bevorzugt größer ist als 5 GC-Fl%, insbesondere größer ist als 10 GC-Fl%. GC-Fl% bedeutet im Rahmen der vorliegenden Erfindung GC-Flächen-%.

Die Hydrierung kann in Substanz oder in einer Flüssigkeit durchgeführt werden.

Geeignete Lösungsmittel sind beispielsweise $C_1$- bis $C_4$-Alkohole, wie Methanol oder Ethanol, $C_4$- bis $C_{12}$-Dialkylether, wie Diethylether oder tert-Butylmethylether, oder cyclische $C_4$- bis $C_{12}$-Ether, wie Tetrahydrofuran oder Dioxan, oder Kohlenwasserstoffe, wie Pentan, Hexan, Heptan, Octan, Cyclohexan. Geeignete Lösungsmittel können auch Mischungen der vorstehend genannten Flüssigkeiten sein. In einer bevorzugten Ausführungsform ist das Lösungsmittel ein Produkt der Hydrierung.

Die Aufarbeitung der Hydrierausträge erfolgt vorzugsweise destillativ. Dabei werden die zugesetzten Alkaliverbindungen als Sumpfprodukte abgetrennt Erfindungsgemäß wird die Hydrierung in Abwesenheit von Ammoniak durchgeführt. Der Druck beträgt bei der Hydrierung vorzugsweise < 85 bar. Erfolgt hingegen die Hydrierung in Gegenwart von Ammoniak kann ebenfalls eine zusätzliche Verbesserung hinsichtlich des Nitrilumsalzes sowie der Selektivität zum primären Amin beobachtet werden. Außerdem trägt Ammoniak zur Abführung der Hydrierwärme bei.

Weiterhin ist es erfindungsgemäß bevorzugt, dass die Vorrichtung (V1) ein Reaktor ist, vorzugsweise beträgt die Querschnittsbelastung 5 bis 50 kg Durchflussmasse pro $m^2$ Querschnittsfläche des Reaktors und Sekunde.

Nachfolgend wird die vorliegende Erfindung anhand von Beispielen verdeutlicht.

Beispiel 1:

Hydrierung von Dimethylaminopropionitril (DMAPN) zu Dimethylaminopropylamin (DMAPA) in Gegenwart eines Kobalt-Vollkontaktkatalysators unter wiederkehrender Zuführung von wässriger Lithiumhydroxid-Lösung.

a) Katalysatorherstellung und Aktivierung

Als Katalysator wird ein Kobalt-Vollkontakt der Zusammensetzung 90,4 Gew.-% Kobalt, 5,1 Gew.-% Mangan, 0,3 Gew.-% Natrium und 3,1 Gew.-% Phosphor (Strangdurchmesser 2mm) verwendet, der nach Beispiel 1 von EP-A 636 409 hergestellt wird. 40,2 g dieses Katalysators werden in einen Hydrierreaktor eingefüllt und in 12 Stunden unter Zufuhr von 25 Nl Wasserstoff pro Stunde auf 280 °C aufgeheizt, 12 Stunden bei dieser Temperatur unter Zuleiten von 25 Nl Wasserstoff pro Stunde gehalten und unter Wasserstoff abgekühlt.

b) Hydrierung von DMAPN

Die Hydrierung wird mit dem vorstehend beschriebenen Katalysator als Festbettkatalysator in einem Rohrreaktor aus Edelstahl (Innenrohr 1.4571) in Rieselfahrweise durchgeführt, der eine Höhe von einem Meter und einen Durchmesser von 0,6 cm besitzt. Der Reaktionsaustrag der Hydrierung wird gekühlt, entspannt, zum Teil ausgeschleust und zum Teil in den Reaktor zurückgeführt.

Als Einsatzstoff für die Hydrierung wird technisches DMAPN verwendet, das Dimethylamin und Wasser enthält. Der Wassergehalt beträgt laut GC-Analyse 0,3 % bis 3,6 Flächen-%, der Gehalt an Dimethylamin 1,4 bis 2,5 Flächen-% (GC-Säule: 60 m CP Volamine/WCOT fused Silica 0,32; Temperaturprogramm: 50 °C - 10 min - 15 °C/min. - 240 °C - 30 min).

Die Hydrierung wird insgesamt 4000 Stunden lang durchgeführt, zunächst 149 Stunden lang ohne Zugabe von wässriger LiOH-Lösung, dann 3800 Stunden lang unter zeitweiliger Zugabe von LiOH-Lösung. In Tabelle 1 sind die Zugabezeiten und die LiOH-Mengen zusammengestellt, Tabelle 2 zeigt die Lithiumkonzentration im Reaktoraustrag.

Tabelle 1: Dosierung von Lithiumhydroxid

| Beginn [h] | Ende [h] | Dauer [h][1] | Konzentration[2] | Menge [g/h][3] | DMAPN [g/h] | Belast ung[4] [kg/lh] | ppm [LiOH/DMAPN][5] | ppm [Li/DMAPN][6] | LiOH absolut [g][7] |
|---|---|---|---|---|---|---|---|---|---|
| 149 | 320 | 171 | 1,00% | 0,0665 | 19,4 | 1,00 | 34,29 | 9,94 | 0,1138 |
| 794 | 915 | 121 | 1,00% | 0,0665 | 19,4 | 1,00 | 34,29 | 9,94 | 0,0803 |
| 1155 | 1270 | 115 | 1,00% | 0,0665 | 19,4 | 1,00 | 34,29 | 9,94 | 0,0762 |
| 1511 | 1845 | 334 | 0,10% | 0,3326 | 19,4 | 1,00 | 17,14 | 4,97 | 0,1110 |
| 1845 | 1968 | 123 | 0,20% | 0,3326 | 19,4 | 1,00 | 34,29 | 9,94 | 0,0820 |
| 1973 | 2159 | 186 | 0,50% | 0,3326 | 19,4 | 1,00 | 85,71 | 24,85 | 0,3097 |
| 2159 | 2181 | 22 | 1,00% | 0,3326 | 19,4 | 1,00 | 171,43 | 49,70 | 0,0726 |
| 2304 | 2379 | 75 | 1,00% | 0,3326 | 19,4 | 1,00 | 171,43 | 49,70 | 0,2494 |
| 2449 | 2541 | 93 | 1,00% | 0,3326 | 19,4 | 1,00 | 171,43 | 49,70 | 0,3079 |
| 2541 | 2668 | 127 | 1,00% | 0,3326 | 19,4 | 0,95 | 171,43 | 49,70 | 0,4208 |
| 2668 | 3116 | 448 | 1,00% | 0,3497 | 20,4 | 1,00 | 171,43 | 49,70 | 1,5678 |
| 3311 | 3621 | 311 | 1,00% | 0,3497 | 20,4 | 1,00 | 171,43 | 49,70 | 1,0863 |

1 Zugabedauer der wässrigen Lithiumhydroxid-Lösung
2 Lithiumhydroxid-Konzentration der wässrigen Lithiumhydroxid-Lösung
3 Menge der wässrigen Lithiumhydroxid-Lösung
4 Kilogramm DMAPN / Liter Katalysator und Stunde
5 Verhältnis von Lithiumhydroxid zu DMAPN (g/g)
6 Verhältnis von Lithium zu DMAPN (g/g)
7 Absolute Menge an Lithiumhydroxid, die innerhalb des jeweiligen Zugabeintervalls in den Reaktoren gefahren wurde.

Gestartet wird die Hydrierung durch Zupumpen von 200 ml rohem DMAPA in den Reaktor.

Die Hydriertemperatur beträgt 90 bis 110°C, der Druck zunächst 700 Stunden lang 85 bar, dann den Rest der Laufzeit 50 bar.

Die Katalysator-Belastung beträgt während der gesamten Laufzeit 1 bis 1,1 kg DMAPN pro Liter Katalysator und Stunde. Die Querschnittsbelastung beträgt 41 bis 42 kg pro $m^2$ und Stunde. Um diese Werte zu erreichen, wird ein Teil des Hydrieraustrags nach Entspannung in den Reaktor zurückgefahren.

Zwischen 2544 und 3500 Stunden Laufzeit wird mit DMAPN gearbeitet, das 5 Gew.-% Wasser enthält.

Während der ersten 149 Stunden Hydrierzeit ohne Zusatz von wässriger LiOH-Lösung enthält der Hydrieraustrag nur 87 bis 90 % DMAPA, 11 bis 13 % Bis-DMAPA und 0,2 % bis 0,3 % DMAPN (GC-Flächen-%), vergleiche auch Vergleichsbeispiel 2, Fahrweise ohne Ammoniak.

Nach 149 Stunden wird mit der ersten Dosierung von wässriger LiOH-Lösung begonnen (Dosierzeiträume in Tabelle 1 angegeben). Nach Zugabe von wässriger LiOH-Lösung beträgt der DMAPN- Umsatz ab 200 Stunden nach Versuchsbeginn während der restlichen 3500 Stunden mindestens 99,4 %. Die DMAPA-Ausbeuten betragen dabei 99,1 bis 99,5 %. Als Nebenprodukt tritt lediglich Bis-DMAPA in Mengen von 0,2 bis 0,9 % auf.

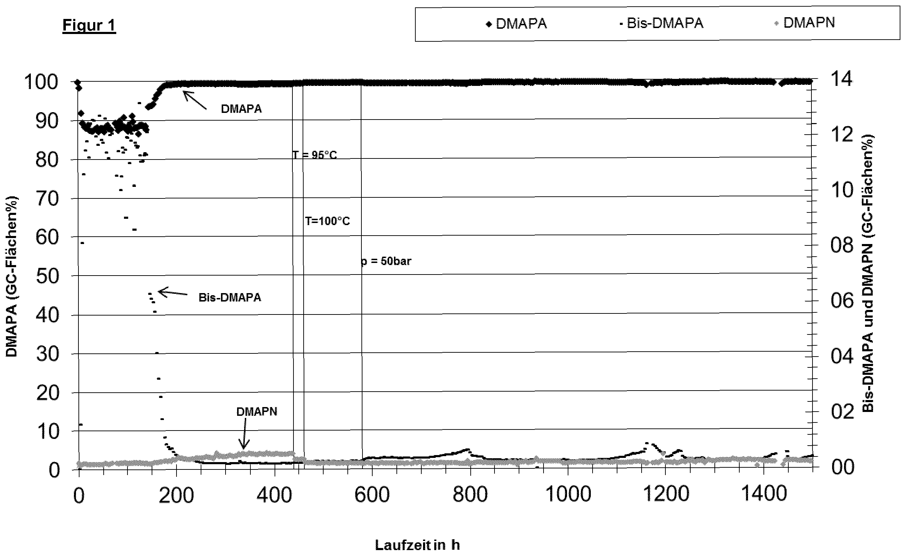

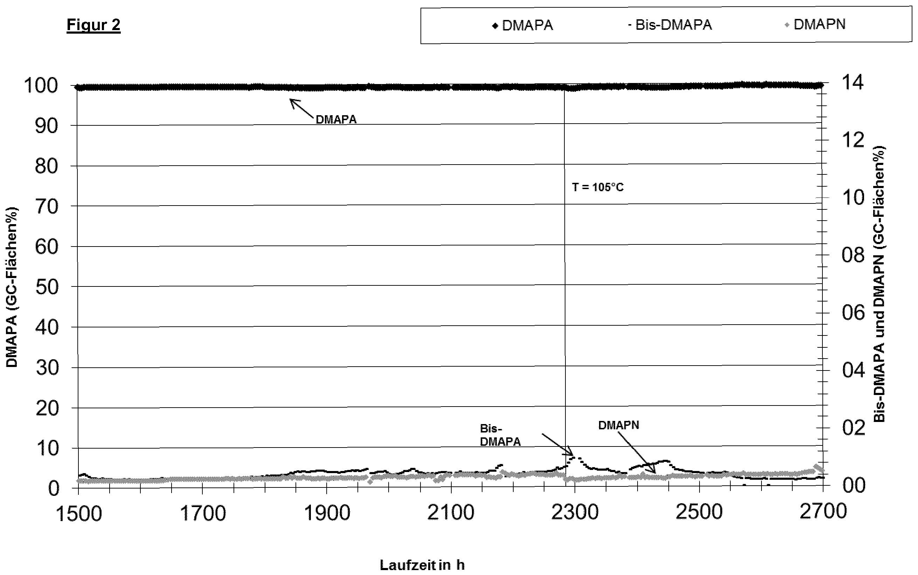

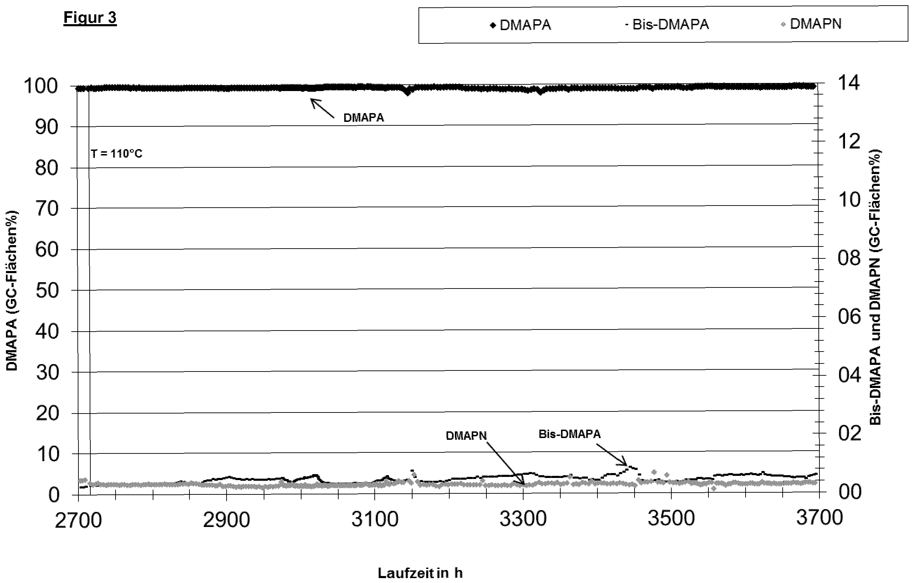

Wie Beispiel 1 zeigt, lassen sich ansteigende oder hohe Bis-DMAPA-Werte während der Fahrweise ohne LiOH-Dosierung durch LiOH-Zugabe wieder auf ein niedrigeres Niveau absenken. Den Figuren 1 bis 3 können hierzu die Konzentrationen an Edukt (DMAPN), Lithiumhydroxid, Produkt (DMAPA) sowie Nebenprodukt (Bis-DMAPA) gemessen am Reaktionsaustrag über die gesamte Versuchsdauer von Beispiel 1 entnommen werden.

Beispiel 1 zeigt weiterhin, dass die positiven Effekte mit geringen LiOH-Mengen erzielbar sind, dass mit hohen Katalysator-Belastungen bei hohen KatalysatorStandzeiten gearbeitet werden kann und dass Wasser im Hydriergemisch nicht stört. Außerdem werden hohe DMAPN-Umsätze und DMAPA-Ausbeuten erzielt, und es kann in Abwesenheit von Ammoniak gearbeitet werden.

Tabelle 2: Bestimmung der Lithiumkonzentration im Austrag

| **Laufzeit [h]** | **Lithiumgehalt Austrag [ppm]** |
|---|---|
| 172 | 14,0 |
| 246 | 7,0 |
| 270 | 17,0 |
| 294 | 10,0 |
| 316 | 12,0 |
| 342 | 2,8 |
| 414 | 1,4 |
| 438 | 1,6 |
| 486 | 1,1 |
| 510 | 0,9 |
| 580 | 1,1 |
| 654 | 0,8 |
| 772 | 0,7 |
| 172 | 14,0 |
| 825 | 22,0 |
| 846 | 17,0 |
| 963 | 2,4 |
| 988 | 2,2 |
| 1007 | 3,0 |
| 1031 | 2,4 |
| 1127 | 1,6 |
| 1201 | 20,0 |

(fortgesetzt)

| Laufzeit [h] | Lithiumgehalt Austrag [ppm] |
|---|---|
| 1609 | 16,0 |
| 1775 | 2,0 |
| 1825 | 1,8 |
| 1871 | 1,8 |
| 1967 | 8,9 |
| 2039 | 1,8 |
| 2117 | 2,8 |
| 2188 | 74,0 |
| 2303 | 3,0 |
| 2351 | 5,0 |
| 2447 | 2,7 |
| 2543 | 10,0 |
| 2615 | 9,0 |
| 2669 | 11,0 |
| 2711 | 11,0 |
| 2813 | 9,0 |
| 3005 | 10,0 |
| 3052 | 9,0 |
| 3143 | 14,0 |
| 3215 | 8,0 |
| 3293 | 4,0 |
| 3337 | 6,0 |
| 3509 | 11,0 |
| 3629 | 11,0 |

Vergleichsbeispiel 2:

Hydrierung von DMAPN zu DMAPA in Gegenwart eines Kobalt-Kontaktkatalysators unter anfänglicher einmaliger Tränkung des Katalysators mit wässriger LiOH-Lösung.

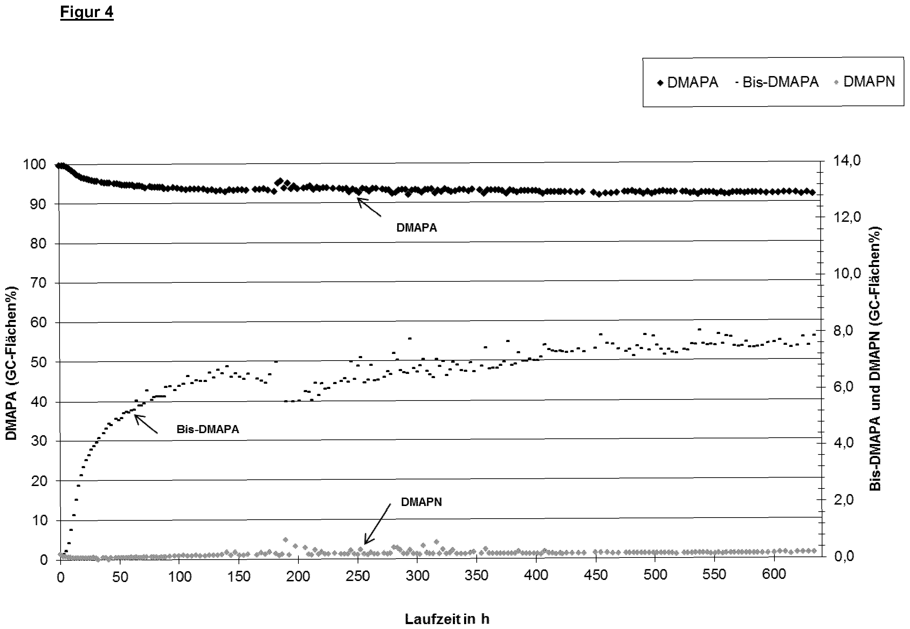

40,2 g der in Beispiel 1 verwendeten Katalysatorcharge werden wie beschrieben aktiviert und der Katalysator in der Anlage wird mit etwa 400 g einer 10%igen wässrigen LiOH-Lösung im geraden Durchgang von unten nach oben durchspült (1ml/min). Etwa 7 Stunden nach Beginn des Zulaufs an LiOH-Lösung wird zusätzlich der Flüssigkeitskreislauf mit einer Rückführmenge von 1000 g/l für 2 Stunden eingeschaltet, anschließend wird die Rückführung und die LiOH-Dosierung wieder abgeschaltet. Im Anschluss werden die gewünschten Reaktionsbedingungen eingestellt (90 °C, Wasserstoff) und die Anlage wird mit DMAPA angefahren. Es wird eine Flüssigkeitsrückführung von 814 g/h und ein DMAPN-Zulauf von 19,4 g/h eingestellt. Der so vorbehandelte Katalysator wird, wie in Beispiel 1 beschrieben, bei 90 °C und 85 bar für die Hydrierung von DMAPN verwendet. Die Konzentration an Lithium im Reaktionsaustrag wird regelmäßig bestimmt. Nach 500 Stunden Reaktionszeit besteht der Hydrieraustrag zu rund 92 % aus DMAPA, rund 8 % aus Bis-DMAPA und weniger als rund 0,5 % aus DMAPN (GC-Flächen-%). Sinngemäß zu den Figuren 1 bis 3 gemäß Beispiel 1 sind die entsprechenden Werte für Vergleichsbeispiel 2 in Figur 4 bildlich dargestellt.

Vergleichsbeispiel 2 zeigt, dass bei Kobalt-Vollkontaktkatalysatoren im Festbett im Gegensatz zu Raney-Cobalt (Katalysator in Suspensionsfahrweise; siehe beispielsweise Beispiel 50 aus EP-A 913 388) eine Vorbehandlung des Katalysators mit LiOH keine Selektivitätssteigerung bewirkt. Durch die regelmäßige Bestimmung von Lithium im Reaktionsaustrag zeigt sich, dass das Lithium kontinuierlich aus der Anlage und von dem Katalysator gewaschen wird. Dieser Effekt ist in dem Vergleichsbeispiel 2 insbesondere etwa in den ersten 200 Stunden nach Beginn der Hydrierung sichtbar. Die Selektivitätsverringerung bezüglich DMAPA und die Zunahme an Bis-DMAPA korreliert mit der aus dem Reaktor herausgewaschenen Menge an Lithium. Bis zum Versuchsende nach etwa 650 Stunden nimmt die Selektivität bezüglich DMAPA weiter kontinuierlich ab und im Reaktionsaustrag ist noch Lithium nachweisbar.

Vergleichsbeispiel 3:

Hydrierung von DMAPN zu DMAPA in Gegenwart eines Kobalt-Vollkontaktkatalysators sowie von Ammoniak bzw. in Abwesenheit von Ammoniak (ohne Lithiumhydroxid-Zugabe)
40,2 g der in Beispiel 1 verwendeten Katalysatorcharge werden wie beschrieben aktiviert. Analog Beispiel 1 wird die Anlage zunächst mit 200 ml rohem DMAPA angefahren. Es wird eine Temperatur von 90 °C und ein Druck von 85 bar eingestellt und während der Versuchsdauer gehalten. Kontinuierlich werden 16 g/h Ammoniak und 25 Nl/h Wasserstoff zudosiert, die Hydrierung wird durch Zufuhr von 19.4 g/h DMAPN gestartet.

Die Katalysator-Belastung beträgt während der gesamten Laufzeit 1 bis 1,1 kg DMAPN pro Liter Katalysator und Stunde. Die Querschnittsbelastung beträgt 34 bis 42 kg pro $m^2$ und Stunde. Um diese Werte zu erreichen, wird ein Teil des Hydrieraustrags nach Entspannung in den Reaktor zurückgefahren. Nach 301 Stunden Reaktionszeit besteht der Hydrieraustrag zu 97,8 % aus DMAPA, weniger als rund 0,3 % aus DMAPN, Bis-DMAPA ist nicht enthalten (GC-Flächen-%).

Nach 312 Stunden wird unter ansonsten unveränderten Versuchsbedingungen die Ammoniakzufuhr unterbrochen. Die erzielte Selektivität zu DMAPA ist in Abwesenheit von Ammoniak deutlich geringer. Nach 442 Stunden Reaktionszeit besteht der Hydrieraustrag zu 85,9 % aus DMAPA, weniger als rund 0,3 % aus DMAPN und zu 11,1 % aus Bis-DMAPA (GC-Flächen-%).

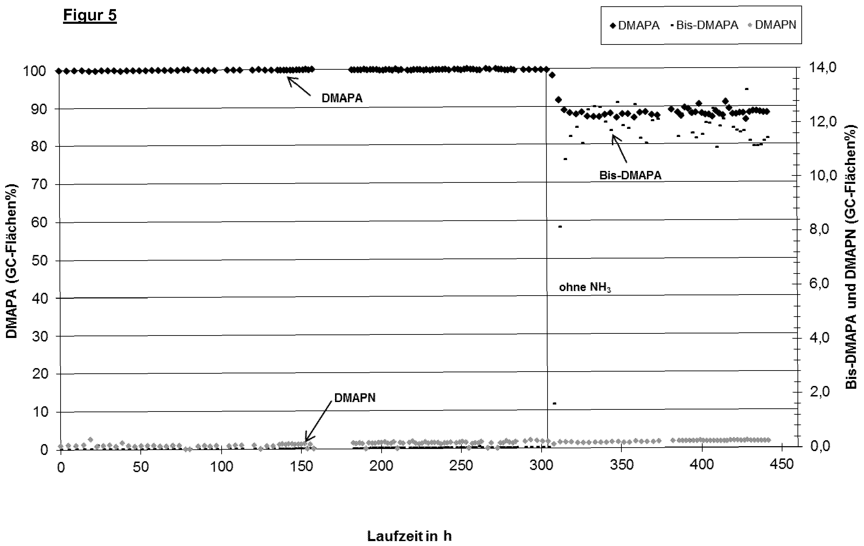

Vergleichsbeispiel 3 zeigt, dass an dem untersuchten Kobalt-Vollkontaktkatalysator in Abwesenheit von Ammoniak bei 85 bar große Mengen an Bis-DMAPA gebildet werden und die Selektivität zu DMAPA gering ist. In Gegenwart von Ammoniak ist aufgrund des Dampfdrucks von Ammoniak eine deutliche Absenkung des Reaktionsdrucks nicht möglich. Die entsprechenden Konzentrationswerte gemäß Vergleichsbeispiel 3 sind analog zu den vorangegangenen Beispielen in Figur 5 bildlich dargestellt.

## Patentansprüche

1. Verfahren zur Herstellung von primären Aminen, **dadurch gekennzeichnet, dass** mindestens ein Nitril in einer Vorrichtung (V1) in Gegenwart eines Kobalt-Vollkontaktkatalysators unter Erhalt von mindestens einem primären Amin hydriert wird, wobei in die Vorrichtung (V1) wiederkehrend oder kontinuierlich mindestens eine Verbindung (I) zugegeben wird und die Verbindung (I) mindestens eine Komponente ausgewählt aus Alkalimetall umfasst, wobei die Hydrierung in Abwesenheit von Ammoniak durchgeführt wird.

2. Verfahren gemäß Anspruch 1, **dadurch gekennzeichnet, dass** die Verbindung (I) in Form eines Oxids, eines Hydroxids und/oder eines Salzes eingesetzt wird und/oder dass die Verbindung (I) als wässrige Lösung eingesetzt wird.

3. Verfahren gemäß Anspruch 2, **dadurch gekennzeichnet, dass** eine wässrige Lösung eines Oxids, eines Hydroxids oder eines Salzes des Lithiums, Kaliums und/oder Caesiums verwendet wird, bevorzugt eine wässrige Lösung von Lithiumhydroxid.

4. Verfahren gemäß einem der Ansprüche 1 bis 3, **dadurch gekennzeichnet, dass**

   i) das zur Hydrierung eingesetzte Nitril 0,01 bis 10 Gew.-% Wasser enthält, und/oder
   ii) die Verbindung (I) in die Vorrichtung (V1) zugegeben wird, sobald der Anteil an als Nebenprodukt gebildetem sekundären Amin größer ist als 0,5 GC-Fl%, und/oder
   iii) in die Vorrichtung (V1) dem Hydriergemisch über die Zugabe der Verbindung (I) 0,01 bis 500 ppm Alkalimetall, bezogen auf g-Atome Nitril in der Vorrichtung (V1), zugegeben werden, vorzugsweise werden in die Vorrichtung (V1) dem Hydriergemisch über die Zugabe der Verbindung (I) 0,01 bis 500 ppm Alkalimetall, bezogen auf g-Atome Nitril in der Vorrichtung (V1), zugegeben, sobald der Anteil an als Nebenprodukt gebildetem sekundären Amin in der Vorrichtung (V1) größer ist als 0,5 GC-Fl%, bei einer Katalysatorbelastung von 0,01 bis 10 kg Edukt pro L Katalysator und Stunde.

5. Verfahren gemäß einem der Ansprüche 1 bis 4, **dadurch gekennzeichnet, dass** die Vorrichtung (V1) ein Reaktor ist, vorzugsweise beträgt die Querschnittsbelastung 5 bis 50 kg Durchflussmasse pro $m^2$ Querschnittsfläche des Reaktors und Sekunde.

6. Verfahren gemäß einem der Ansprüche 1 bis 5, **dadurch gekennzeichnet, dass** die Hydrierung bei Drucken von 1 bis 300 bar durchgeführt wird.

7. Verfahren gemäß einem der Ansprüche 1 bis 6, **dadurch gekennzeichnet, dass** der Kobalt-Vollkontaktkatalysator in der Vorrichtung (V1) als Festbettkatalysator verwendet wird.

8. Verfahren gemäß einem der Ansprüche 1 bis 7, **dadurch gekennzeichnet, dass** das Nitril ein aliphatisches Mono-, Di- oder Trinitril, ein cycloaliphatisches Mono- oder Dinitril, ein alpha-, beta- oder omega- Aminonitril oder ein Alkoxynitril ist.

9. Verfahren gemäß einem der Ansprüche 1 bis 8, **dadurch gekennzeichnet, dass**

   i) N,N-Dimethylaminopropionitril (DMAPN) zur Herstellung von N,N-Dimethylaminopropylamin (DMAPA) eingesetzt wird, oder
   ii) Isophoronnitrilimin zur Herstellung von Isophorondiamin eingesetzt wird, oder
   iii) Adipodinitril (ADN) zur Herstellung von Hexamethylendiamin (HMD) oder zur Herstellung von 6-Aminocapronitril (6-ACN) und HMD eingesetzt wird.

10. Verfahren gemäß einem der Ansprüche 1 bis 9, **dadurch gekennzeichnet, dass** der Kobalt-Vollkontaktkatalysator zu mindestens 70 Gew.-%, vorzugsweise zu mindestens 80 Gew.-%, besonders bevorzugt zu mindestens 90 Gew.-% und ganz besonders bevorzugt zu mindestens 95 Gew.-% aus katalytisch aktiver Masse besteht.

11. Verfahren gemäß einem der Ansprüche 1 bis 10, **dadurch gekennzeichnet, dass** der Kobalt-Vollkontaktkatalysator als katalytisch aktive Masse die Komponente i) und gegebenenfalls die Komponente ii) und/oder iii) umfasst, mit:

    i) Kobalt (Co),
    ii) gegebenenfalls mindestens ein weiteres Element ausgewählt aus Eisen (Fe), Nickel (Ni), Ruthenium (Ru), Rhodium (Rh), Palladium (Pd), Osmium (Os), Iridium (Ir) oder Platin (Pt), und/oder
    iii) gegebenenfalls mindestens einen Promotor ausgewählt aus Chrom (Cr), Mangan (Mn), Molybdän (Mo), Titan (Ti), Zink (Zn), Zinn (Sn), Alkalimetalle, Erdalkalimetalle, Seltene Erdmetalle oder Phosphor (P).

12. Verfahren gemäß einem der Ansprüche 1 bis 11, **dadurch gekennzeichnet, dass** die katalytische aktive Masse des Kobalt-Vollkontaktkatalysator aus 55 bis 98 Gew.-% Kobalt, 0,2 bis 15 Gew.-% Phosphor, 0,2 bis 15 Gew.-% Mangan und 0,2 bis 15 Gew.-% Alkali (berechnet als CoO, $H_3PO_4$, $MnO_2$ oder Alkalioxid vor der Reduktion mit Wasserstoff) besteht, vorzugsweise wird der Kobalt-Vollkontaktkatalysator hergestellt, indem die Katalysatormasse in einem ersten Schritt bei Endtemperaturen von 550 bis 750 °C und in einem zweiten Schritt bei Endtemperaturen von 800 bis 1000 °C calciniert wird.

13. Verfahren gemäß einem der Ansprüche 1 bis 12, **dadurch gekennzeichnet, dass** der Kobalt-Vollkontaktkatalysator

    i) eine Oberfläche von maximal 50 $m^2$/g aufweist, bevorzugt von maximal 40 $m^2$/g, besonders bevorzugt von maximal 30 $m^2$/g und/oder
    ii) der Anteil an Trägermaterial maximal 10 Gew.-% (bezogen auf die Katalysatorgesamtmasse), bevorzugt maximal 5 Gew.-%, besonders bevorzugt maximal 1 Gew.-%, ist, insbesondere ist der Kobalt-Vollkontaktkatalysator frei von Trägermaterial.

14. Verfahren gemäß einem der Ansprüche 1 bis 13, **dadurch gekennzeichnet, dass** ein Strom (S1) aus der Vorrichtung (V1) ausgeleitet wird, wobei der Strom (S1) das primäre Amin enthält und der Strom (S1) zumindest teilweise in die Vorrichtung (V1) rückgeleitet wird,

    vorzugsweise wird die Verbindung (I) zunächst in den rückgeleiteten Teil des Stromes (S1) zugegeben und als Bestandteil des Stroms (S1) in die Vorrichtung (V1) eingespeist,
    besonders bevorzugt wird die Verbindung (I) als wässrige Lösung in den rückgeleiteten Teil des Strom (S1) zugegeben.

## Claims

1. A process for preparing primary amines, which comprises hydrogenating at least one nitrile in an apparatus (V1) in the presence of an unsupported cobalt catalyst to obtain at least one primary amine, with recurrent or continuous addition of at least one compound (I) to the apparatus (V1), said compound (I) comprising at least one component selected from alkali metal, wherein the hydrogenation is conducted in the absence of ammonia.

2. The process according to claim 1, wherein said compound (I) is used in the form of an oxide, a hydroxide and/or a salt and/or said compound (I) is used as an aqueous solution.

3. The process according to claim 2, wherein an aqueous solution of an oxide, a hydroxide or a salt of lithium, potassium and/or cesium is used, preferably an aqueous solution of lithium hydroxide.

4. The process according to any of claims 1 to 3, wherein

   i) the nitrile used for hydrogenation comprises 0.01% to 10% by weight of water, and/or
   ii) the compound (I) is added to the apparatus (V1) as soon as the proportion of secondary amine formed as by-product is greater than 0.5 GC area%, and/or
   iii) added to the hydrogenation mixture in the apparatus (V1) via the addition of the compound (I) is 0.01 to 500 ppm of alkali metal, based on g-atoms of nitrile in the apparatus (V1), preference being given to adding to the hydrogenation mixture in the apparatus (V1) via the addition of the compound (I) 0.01 to 500 ppm of alkali metal, based on g-atoms of nitrile in the apparatus (VI), as soon as the proportion of secondary amine formed as by-product in the apparatus (V1) is greater than 0.5 GC area%, at a catalyst space velocity of 0.01 to 10 kg of reactant per L of catalyst and hour.

5. The process according to any of claims 1 to 4, wherein the apparatus (V1) is a reactor, the superficial velocity preferably being 5 to 50 kg of mass flow per $m^2$ of cross-sectional reactor area and second.

6. The process according to any of claims 1 to 5, wherein the hydrogenation is conducted at pressures of 1 to 300 bar.

7. The process according to any of claims 1 to 6, wherein the unsupported cobalt catalyst is used in the apparatus (V1) as a fixed bed catalyst.

8. The process according to any of claims 1 to 7, wherein the nitrile is an aliphatic mono-, di- or trinitrile, a cycloaliphatic mono- or dinitrile, an alpha-, beta- or omega-aminonitrile or an alkoxynitrile.

9. The process according to any of claims 1 to 8, wherein

   i) N,N-dimethylaminopropionitrile (DMAPN) is used to prepare N,N-dimethylaminopropylamine (DMAPA), or
   ii) isophoronenitrileimine is used to prepare isophoronediamine, or
   iii) adiponitrile (ADN) is used to prepare hexamethylenediamine (HMD) or to prepare 6-aminocapronitrile (6-ACN) and HMD.

10. The process according to any of claims 1 to 9, wherein the unsupported cobalt catalyst consists of catalytically active composition to an extent of at least 70% by weight, preferably to an extent of at least 80% by weight, more preferably to an extent of at least 90% by weight and most preferably to an extent of at least 95% by weight.

11. The process according to any of claims 1 to 10, wherein the unsupported cobalt catalyst comprises, as catalytically active composition, component i) and optionally component ii) and/or iii), with:

    i) cobalt (Co),
    ii) optionally at least one further element selected from iron (Fe), nickel (Ni), ruthenium (Ru), rhodium (Rh), palladium (Pd), osmium (Os), iridium (Ir) and platinum (Pt), and/or
    iii) optionally at least one promoter selected from chromium (Cr), manganese (Mn), molybdenum (Mo), titanium (Ti), zinc (Zn), tin (Sn), alkali metals, alkaline earth metals, rare earth metals and phosphorus (P).

12. The process according to any of claims 1 to 11, wherein the catalytically active composition of the unsupported cobalt catalyst consists of 55% to 98% by weight of cobalt, 0.2% to 15% by weight of phosphorus, 0.2% to 15% by weight of manganese and 0.2% to 15% by weight of alkali metal (calculated as CoO, $H_3PO_4$, $MnO_2$ or alkali metal oxide prior to reduction with hydrogen), preference being given to preparing the unsupported cobalt catalyst by calcining the catalyst composition in a first step at final temperatures of 550 to 750°C and in a second step at final temperatures of 800 to 1000°C.

**13.** The process according to any of claims 1 to 12, wherein the unsupported cobalt catalyst

i) has a surface area of not more than 50 $m^2$/g, preferably of not more than 40 $m^2$/g, more preferably of not more than 30 $m^2$/g, and/or
ii) the proportion of support material is not more than 10% by weight (based on the total mass of the catalyst), preferably not more than 5% by weight, more preferably not more than 1% by weight, the unsupported cobalt catalyst especially being free of support material.

**14.** The process according to any of claims 1 to 13, wherein a stream (S1) which is discharged from the apparatus (V1) comprises the primary amine and is returned at least partly to the apparatus (V1),

preference being given to adding compound (I) at first to the returned portion of stream (S1) and feeding it into the apparatus (V1) as a constituent of stream (S1),
particular preference being given to adding compound (I) as an aqueous solution to the returned portion of stream (S1).

## Revendications

**1.** Procédé pour la préparation d'amines primaires, **caractérisé en ce qu'**au moins un nitrile est hydrogéné dans un dispositif (V1) en présence d'un catalyseur de contact entier au cobalt avec obtention d'au moins une amine primaire, au moins un composé (I) étant ajouté dans le dispositif (V1) de manière récurrente ou de manière continue et le composé (I) comprenant au moins un composant choisi parmi un métal alcalin, l'hydrogénation étant réalisée en l'absence d'ammoniac.

**2.** Procédé selon la revendication 1, **caractérisé en ce que** le composé (I) est utilisé sous forme d'un oxyde, d'un hydroxyde et/ou d'un sel et/ou **en ce que** le composé (I) est utilisé en tant que solution aqueuse.

**3.** Procédé selon la revendication 2, **caractérisé en ce qu'**une solution aqueuse d'un oxyde, d'un hydroxyde ou d'un sel de lithium, de potassium et/ou de césium est utilisée, préférablement une solution aqueuse d'hydroxyde de lithium.

**4.** Procédé selon l'une quelconque des revendication 1 à 3, **caractérisé en ce que**

i) le nitrile utilisé pour l'hydrogénation contient 0,01 à 10 % en poids d'eau, et/ou
ii) le composé (I) est ajouté dans le dispositif (V1) dès que la proportion d'amine secondaire formée comme sous-produit est supérieure à 0,5 % en surface en GC, et/ou
iii) dans le dispositif (V1), 0,01 à 500 ppm de métal alcalin, par rapport aux atomes-grammes de nitrile dans le dispositif (V1) sont ajoutés au mélange d'hydrogénation durant l'ajout du composé (I), de préférence, dans le dispositif (V1), 0,01 à 500 ppm de métal alcalin, par rapport aux atomes-grammes de nitrile dans le dispositif (V1) sont ajoutés au mélange d'hydrogénation durant l'ajout du composé (I), dès que la proportion d'amine secondaire formée comme sous-produit dans le dispositif (V1) est supérieure à 0,5 % en surface en GC, avec une charge de catalyseur de 0,01 à 10 kg d'éduit par L de catalyseur et heure.

**5.** Procédé selon l'une quelconque des revendication 1 à 4, **caractérisé en ce que** le dispositif (V1) est un réacteur, de préférence le chargement de section transversale est de 5 à 50 kg de masse d'écoulement par $m^2$ de surface de section transversale du réacteur et seconde.

**6.** Procédé selon l'une quelconque des revendication 1 à 5, **caractérisé en ce que** l'hydrogénation est réalisée à des pressions de 1 à 300 bars.

**7.** Procédé selon l'une quelconque des revendication 1 à 6, **caractérisé en ce que** le catalyseur de contact entier au cobalt est utilisé dans le dispositif (V1) en tant que catalyseur à lit fixe.

**8.** Procédé selon l'une quelconque des revendication 1 à 7, **caractérisé en ce que** le nitrile est un mononitrile, dinitrile ou trinitrile aliphatique, un mononitrile ou dinitrile cycloaliphatique, un alpha-aminonitrile, bêta-aminonitrile ou oméga-aminonitrile ou un alcoxynitrile.

**9.** Procédé selon l'une quelconque des revendications 1 à 8, **caractérisé en ce que**

i) le N,N-diméthylaminopropionitrile (DMAPN) est utilisé pour la préparation de la N,N-diméthylaminopropylamine (DMAPA), ou
ii) l'isophoronenitrilimine est utilisée pour la préparation de l'isophoronediamine, ou
iii) l'adipodinitrile (ADN) est utilisé pour la préparation de l'hexaméthylènediamine (HMD) ou pour la préparation du 6-aminocapronitrile (6-ACN) et de la HMD.

**10.** Procédé selon l'une quelconque des revendications 1 à 9, **caractérisé en ce que** le catalyseur de contact entier au cobalt est constitué d'au moins 70 % en poids, de préférence d'au moins 80 % en poids, particulièrement préférablement d'au moins 90 % en poids et tout particulièrement préférablement d'au moins 95 % en poids de masse catalytiquement active.

**11.** Procédé selon l'une quelconque des revendications 1 à 10, **caractérisé en ce que** le catalyseur de contact entier au cobalt comprend, en tant que masse catalytiquement active, le composant i) et éventuellement le composant ii) et/ou iii), avec :

i) cobalt (Co),
ii) éventuellement au moins un élément supplémentaire choisi parmi le fer (Fe), le nickel (Ni), le ruthénium (Ru), le rhodium (Rh), le palladium (Pd), l'osmium (Os), l'iridium (Ir) et le platine (Pt), et/ou
iii) éventuellement au moins un promoteur choisi parmi le chrome (Cr), le manganèse (Mn), le molybdène (Mo), le titane (Ti), le zinc (Zn), l'étain (Sn), des métaux alcalins, des métaux alcalino-terreux, des métaux des terres rares et le phosphore (P).

**12.** Procédé selon l'une quelconque des revendications 1 à 11, **caractérisé en ce que** la masse catalytiquement active du catalyseur de contact entier au cobalt est constituée de 55 à 98 % en poids de cobalt, 0,2 à 15 % en poids de phosphore, 0,2 à 15 % en poids de manganèse et 0,2 à 15 % en poids d'alcali (calculés comme CoO, $H_3PO_4$, $MnO_2$ ou oxyde d'alcali avant la réduction avec l'hydrogène), de préférence le catalyseur de contact entier au cobalt est préparé en calcinant la masse de catalyseur dans une première étape à des températures finales de 550 à 750 °C et dans une deuxième étape à des températures finales de 800 à 1 000 °C.

**13.** Procédé selon l'une quelconque des revendications 1 à 12, **caractérisé en ce que**

i) le catalyseur de contact entier au cobalt présente une surface de maximum 50 $m^2/g$, préférablement de maximum 40 $m^2/g$, particulièrement préférablement de maximum 30 $m^2/g$ et/ou
ii) la proportion de matériau de support est de maximum 10 % en poids (par rapport à la masse totale de catalyseur), préférablement de maximum 5 % en poids, particulièrement préférablement de maximum 1 % en poids, en particulier le catalyseur de contact entier au cobalt est exempt de matériau de support.

**14.** Procédé selon l'une quelconque des revendications 1 à 13, **caractérisé en ce qu'**un flux (S1) est évacué du dispositif (V1), le flux (S1) contenant l'amine primaire et le flux (S1) étant reconduit au moins partiellement dans le dispositif (V1),

de préférence, le composé (I) étant d'abord ajouté dans la partie reconduite du flux (S1) et étant injecté dans le dispositif (V1) en tant qu'ingrédient du flux (S1),
particulièrement préférablement, le composé (I) étant ajouté en tant que solution aqueuse dans la partie reconduite du flux (S1).

DMAPA
Bis-DMAPA
DMAPN
DMAPA (GC-Flächen%)
Bis-DMAPA und DMAPN (GC-Flächen%)
Laufzeit in h
T = 95°C
T=100°C
p = 50bar

Figur 1

Figur 2

◆ DMAPA
- Bis-DMAPA
◆ DMAPN
DMAPA
T = 105°C
Bis-DMAPA
DMAPN
DMAPA (GC-Flächen%)
Bis-DMAPA und DMAPN (GC-Flächen%)
Laufzeit in h
1500
1700
1900
2100
2300
2500
2700
0
10
20
30
40
50
60
70
80
90
100

Figur 3

DMAPA
Bis-DMAPA
DMAPN
T = 110°C
DMAPA (GC-Flächen%)
Bis-DMAPA und DMAPN (GC-Flächen%)
Laufzeit in h
2700
2900
3100
3300
3500
3700
100
90
80
70
60
50
40
30
20
10
0

Figur 4

DMAPA
Bis-DMAPA
DMAPN
Bis-DMAPA und DMAPN (GC-Flächen%)
14,0
12,0
10,0
8,0
6,0
4,0
2,0
0,0
DMAPA (GC-Flächen%)
100
90
80
70
60
50
40
30
20
10
0
0
50
100
150
200
250
300
350
400
450
500
550
600
Laufzeit in h

DMAPA
Bis-DMAPA
DMAPN
Bis-DMAPA
ohne $NH_3$
DMAPA
DMAPN
DMAPA (GC-Flächen%)
Bis-DMAPA und DMAPN (GC-Flächen%)
Laufzeit in h
100
90
80
70
60
50
40
30
20
10
0
14,0
12,0
10,0
8,0
6,0
4,0
2,0
0,0
0
50
100
150
200
250
300
350
400
450

Figur 5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 913388 A
- WO 2003070688 A
- WO 2007104663 A1
- WO 2007104663 A
- WO 2006077233 A1
- US 5569653 A
- FR 1530809 A
- US 5869653 A
- WO 2010089346 A2
- EP 0424738 A2
- EP 0636409 A
- DE 3403377 A1
- EP 0742045 A
- EP 963975 A
- EP 445589 B1
- EP 1106600 A2
- EP 636409 A


### In der Beschreibung aufgeführte Nicht-Patentliteratur


- Fixed-Bed Reactors. *Ullmann's Encyclopedia of Industrial Chemistry,* 15. Juni 2000
- **A. B. STILES.** Catalyst Manufacture - Laboratory and Commercial Preparations. Marcel Dekker, 1983
- Catalysis and Catalysts. Ullmann's Encyclopedia Electronic Release. 2000, 28-32
- **ERTL ; KNÖZINGER ; WEITKAMP.** Handbook of Heterogenoeous Catalysis. VCH Weinheim, 1997, 98
- **A. B. STILES.** Catalyst Manufacture. Marcel Dekker, Inc, 1983, 15